# EUROPÄISCHE PATENTSCHRIFT

(11) **EP 1 031 626 B1**
(45) Veröffentlichungstag und Bekanntmachung des Hinweises auf die Patenterteilung: **26.01.2011**
(21) Anmeldenummer: 00103816.5
(22) Anmeldetag: 23.02.2000
(51) Int. Cl.: C12N 15/10, C07D 295/037, C07C 211/63

(54) **Verfahren zur Stabilisierung und/oder Isolierung von Nukleinsäuren**
Method for stabilising and/or isolating nucleic acids
Procédé pour la stabilisation et/ou l'isolement d'acides nucléiques

(30) Priorität: 23.02.1999 EP 99103457
(43) Veröffentlichungstag der Anmeldung: 30.08.2000
(73) Patentinhaber: QIAGEN GmbH, 40724 Hilden (DE)
(72) Erfinder: Erbacher, Christoph, 42781 Haan (DE); Bastian, Helge, 40597 Düsseldorf-Benrath (DE); Wyrich, Ralf, 41542 Dormagen (DE); Oelmüller, Uwe, 40699 Erkrath (DE); Manz, Thomas, 40237 Düsseldorf (DE)
(74) Vertreter: Zimmermann, Gerd Heinrich

(56) Entgegenhaltungen:
- EP-A- 0 773 295
- WO-A-94/18156
- WO-A-98/19709
- DE-A- 2 154 278
- US-A- 5 010 183
- DATABASE WPI Section Ch, Week 8445 Derwent Publications Ltd., London, GB; Class B04, AN 84-281416 XP002111373 & SU 1 081 171 A (KIEV DOTORS TRAIN), 23. März 1984 (1984-03-23)
- SYKORA ET AL: "Method of plasmid removal from bacterial cells" CHEMICAL ABSTRACTS, Bd. 7, Nr. 115, 19. August 1991 (1991-08-19), XP002111378 Columbus, Ohio, us & CS 266 103 A (SZECH)
- SYKORA ET AL: "Elimination of plasmids pKM 101 and F'lac from Salmonella typhimurium and Escherichia coli by bisammonium salt. The effect of outer membrane pattern" CHEMICAL ABSTRACTS, Bd. 25, Nr. 115, 23. Dezember 1991 (1991-12-23), XP002111379 Columbus, Ohio, US & FOLIA MICROBIOL. (PRAGUE) (1991), 36(3),240-5; CODEN: FOMIAZ; ISSN: 0015-5632,
- HORNIAK ET AL: "Interaction of DNA and DNA-calcium-model phosphatidylcholine membrane complex with surface active bisammonium salts" CHEMICAL ABSTRACTS, Bd. 3, Nr. 110, 16. Januar 1989 (1989-01-16), XP002111380 Columbus, Ohio, US & STUD. BIOPHYS. (1988), 124(1), 61-8; CODEN: STBIBN; ISSN: 0081-6337,

## Beschreibung

Die vorliegende Erfindung betrifft ein Verfahren zur Stabilisierung und Isolierung von Nukleinsäuren, wobei eine biologische Probe, die Nukleinsäuren enthält, mit einer kationischen Verbindung in Kontakt gebracht wird.

Es ist seit langem bekannt, daß die genetische Herkunft und funktionelle Aktivität einer Zelle durch Studien ihrer Nukleinsäuren bestimmt und untersucht werden kann. Die Analysen der Nukleinsäuren ermöglichen den direkten Zugriff auf die Ursache der Aktivitäten von Zellen. Sie sind somit indirekten, konventionellen Methoden, wie z.B. dem Nachweis von Stoffwechsechselprodukten, potentiell überlegen. Daher ist für die Zukunft mit einer starken Verbreitung von Nukleinsäureanalysen zu rechnen. So werden molekularbiologische Analysen bereits in vielen Bereichen eingesetzt, z.B. in der medizinischen und klinischen Diagnostik, in der Pharmazie bei der Entwicklung und Evaluierung von Arzneimitteln, in der Lebensmittelanalytik sowie bei der Überwachung der Lebensmittelherstellung, in der Agrarwirtschaft bei der Züchtung von Nutzpflanzen und Nutztieren sowie in der Umweltanalytik und in vielen Forschungsgebieten.

Durch die Analyse der RNA, speziell der mRNA in Zellen, lassen sich die Aktivitäten von Genen direkt bestimmen. Die quantitative Analyse von Transkriptmustern (mRNA-Mustern) in Zellen durch moderne molekularbiologische Methoden, wie z.B. Echtzeit-Reverse Transkriptase PCR ("Real-Time RT-PCR") oder Genexpressions-Chip-Analysen ermöglicht z.B. die Erkennung fehlerhaft exprimierter Gene, wodurch z.B. Stoffwechselkrankheiten, Infektionen oder die Entstehung von Krebs erkannt werden können. Die Analyse der DNA aus Zellen durch molekularbiologische Methoden, wie z.B. PCR, RFLP, AFLP oder Sequenzierung ermöglicht z.B. den Nachweis genetischer Defekte oder die Bestimmung des HLA-Typs sowie anderer genetischer Marker.

Die Analyse genomischer DNA und RNA wird auch zum direkten Nachweis von infektiösen Erregern, wie Viren, Bakterien usw. eingesetzt.

Unbedingte Voraussetzung für Nukleinsäureanalytik ist die sofortige Stabilisierung der Nukleinsäuren nach Entnahme der biologischen Probe aus ihrer natürlichen Umgebung. Dies gilt für DNA und RNA, insbesondere für RNA, die nach Entnahme der biologischen Probe sehr schnell abgebaut werden kann. Andererseits kann es nach der Entnahme der biologischen Probe z.B. durch Induktion von Streßgenen auch zur Synthese neuer mRNA-Moleküle kommen, wodurch das Transkriptmuster der Zellen verändert werden kann. Dadurch können nachfolgende Analysen verfälscht werden.

Die Stabilisierung von Nukleinsäuren, insbesondere über längere Zeiträume wie z.B. mehrere Stunden oder Tage bis zu Wochen mit Hilfe von für Routineanalysen geeigneten Mitteln ist bislang kaum möglich. Dies ist sehr nachteilhaft, da z.B. im medizinischen Bereich häufig, etwa in einer Praxis, Nukleinsäure-haltige Proben genommen werden, die erst nach längerer Lagerung und einem Transport in ein Labor weiter untersucht werden können.

In der Zwischenzeit können sich die in den Proben enthaltenen Nukleinsäure verändern oder sogar vollständig zersetzen. Dies beeinflußt natürlich das Ergebnis später durchgeführter Tests massiv oder macht diese gänzlich unmöglich. Für solche Tests werden molekularbiologische Techniken wie bespielsweise PCR, Reversed-Transcription-PCR (RT-PCR), SunRise, LCR, branched-DNA (bDNA), SDA, DNA- und RNA-Chips und Arrays zur Genexpressions- und Mutationsanalytik, differential display Analytik, RFLP, AFLP, cDNA-Synthesen, subtraktive Hybridisierung oder die TaqMan-Technologie und ähnliche Echtzeitquantifizierungsverfahren eingesetzt.

Abgesehen von der Stabilisierung betrifft die vorliegende Erfindung auch die Isolierung von Nukleinsäuren.

Dabei soll die Bezeichnung "Nukleinsäure" in ihrem breitesten Sinn verstanden werden, also Ribonukleinsäuren (RNA) wie auch Desoxyribonukleinsäuren (DNA) in allen Längen und Konfigurationen, wie Doppelstrang, Einzelstrang, circulär und linear, verzweigt usw. umfassen und alle möglichen Unterarten, wie z.B. monomere Nukleotide, Oligomere, Plasmide, virale und bakterielle DNA und RNA, sowie genomische und nichtgenomische DNA und RNA aus Tier- und Pflanzenzellen oder anderen Eukaryoten, mRNA in prozessierter und unprozessierter Form, tRNA, hn-RNA, rRNA, cDNA usw.

Stabilisierung und Isolierung sind zwei wichtige Schritte in einer Reaktionskaskade, die eine Analyse auf Nukleinsäurebasis darstellt. Diese Kaskade könnte schematisch wie folgt dargestellt werden:

Probennahme -> Stabilisierung -> Nukleinsäure- -> Enzymatische -> Nachweis -> Datenanalyse
Lagerung, isolierung/ Manipulation
Reinigung

Die vorliegende Erfindung befaßt sich mit den hervorgehobenen Schrittendieser Kaskade.

Es gibt eine Reihe von Verfahren zur Isolierung von Nukleinsäuren, wobei die Zelle zerstört wird und die RNA und/oder DNA in Lösung freigesetzt wird. Bekannte Verfahren zur Isolierung von Nukleinsäuren aus komplexen Materialien wie Blut, Serum, Urin oder Faeces umfassen in der Regel die Lyse des biologischen Materials durch ein Detergenz in Gegenwart von Proteinasen, gefolgt von mehreren Extraktionen mit organischen Lösungsmitteln, z.B. Phenol und/oder Chloroform, eine Ethanol-Präzipitation und eine Dialyse der Nukleinsäuren. Solche Verfahren werden z.B. von Chirgwin et al. (Biochem, 18:5294-5299 (1979)), D.M. Wallace in Meth. Enzym., 152:33-41 (1987), (P. Chomczynski und N. Sacchi, Anal. Biochem., 162:156-159 (1987) und "Preparation and Analysis of RNA" in Current Protocols in Molecular Biology, Unit 4.2 (Supplement 14), Herausgeber F.M. Ausubel et al., John Wiley (1991)), (T. Maniatis et al., Molecular Cloning, A Laboratory Manual, Cold Spring Harbour Laboratory (1992)); (L.G. Davis et al. "Guanidine Isothiocyanate Preparation of Total RNA" und "RNA Preparation: Mini Method" in Basic Methods in Molecular Biology, Elsevier, N.Y. Seiten 130-138 (1991)) und in dem US Patent Nr. 4,843,155 für Chomczynski beschrieben.

Es ist ferner bekannt Nukleinsäuren aus verschiedenen Ausgangsmaterialien zu isolieren, indem das Ausgangsmaterial mit einer chaotropen Substanz und einer die Nukleinsäure bindenen, festen Phase gemischt wird. In einem weiteren Schritt wird die feste Phase von der Flüssigkeit getrennt und gewaschen. Falls nötig, können die Nukleinsäuren von der festen Phase eluiert werden (US 5,234,809).

Diese bekannten Verfahren zur Isolierung von Nukleinsäuren aus biologischen Materialien sind oft sehr arbeits- und zeitaufwendig. Die meist relativ große Anzahl von Schritten, die notwendig ist um die Nukleinsäuren aus solchen Ausgangsmaterialien zu reinigen, vergrößern das Risiko einer Übertragung der Nukleinsäuren von Probe zur Probe, wenn gleichzeitig verschiedene klinische Proben verarbeitet werden. Wenn die Nukleinsäure für den nachfolgenden Nachweis der Gegenwart von Nukleinsäuren von z.B. einem pathogenen Organismus durch ein Nukleinsäure-Amplifikationsverfahren isoliert wird, z.B. für die sehr sensitive Polymerase-Kettenreaktion, führt das Risiko einer solchen Übertragung von Nukleinsäuren zwischen verschiedenen Proben zu falschpositiven Resultaten, was natürlich einen ernsten Nachteil darstellt.

In MacFarlane US 5,010,183 und MacFarlane US 5,300,635 werden ausführlich Verfahren zur Isolierung von Nukleinsäuren unter Verwendung kationischer Detergenzien auf der Basis quartärer Ammoniumverbindungen beschrieben. Die in den oben genannten Patenten geschützten Ammoniumverbindungen haben alle die allgemeine Form [N(R)₄]⁺ X⁻, wobei R für verschiedene Alkyl- oder Arylgruppen mit einer unterschiedlichen Anzahl der C-Atome steht und X für ein Gegenion aus einer Gruppe von Carbonsäuren, Sulfat, Phosphat oder einem Halogenid. Außerdem sind hohe g-Zahlen zur Pelletierung der Komplexe aus Nukleinsäure und Detergenz notwendig. Die Isolierung von Nukleinsäuren mit den oben beschriebenen Verfahren ist zwar im Prinzip möglich, jedoch sind hohe Carrier-Mengen und hohe g-Zahlen notwendig.

Die in den oben genannten Patenten beschriebenen Beispiele beziehen sich alle auf die Extraktion von Nukleinsäuren aus Vollblut oder Zellen (menschliche und E.coli). In diesen Probenmaterialien ist eine gewisse Mindestmenge an Nukleinsäuren vorhanden. Z.T. wurde zusätzlich noch tRNA als Carrier zugegeben. Am Beispiel der Aufreinigung von kleinen Mengen (z.B. in niedrigen Kopienzahlen bei viralen Infektionen) von RNA aus zellfreien Probenmaterialien wie Plasma konnte am Beispiel des Tetradecyltrimethylammonium-Oxalats gezeigt werden, daß eine Komplexierung/Pelletierung nur unter Einsatz großer Mengen Carrier RNA (100 µg/ml Plasma) möglich ist. Die Notwendigkeit einer solchen Aufreinigung stellt sich z.B. beim Nachweis viraler RNA in Plasma- oder Serumproben. Die hohen Carriermengen stellen bei einem nachfolgenden Nachweis der viralen RNA mittels RT-PCR ein Problem dar, da die reverse Transkription durch hohe Carrierkonzentrationen inhibiert wird. MacFarlane beschreibt selber auch eine niedrigere Sensitivität des Nachweises von HCV in Plasma (ohne Carrier), verglichen mit Blut (Schmidt et al. J.Med.Virol. 47: 153-160 (1995)). Ohne hohe Nukleinsäuremengen ist die Sensitivität sehr schlecht. Desweiteren beschreibt MacFarlane in US 5,300,635 die Sedimentation der RNA-Detergenz-Komplexe durch Zentrifugation bei hohen g-Zahlen (16000 x g in den Beispielen 4, 5 und 6). Es konnte ebenfalls gezeigt werden, daß eine Zentrifugation bei niedrigeren g-Zahlen nicht ausreicht, um RNATetradecyltrimethylammonium Oxalat-Komplexe aus Plasma zu sedimentieren. Zur Aufreinigung viraler RNA aus großen Volumina Plasma oder Serum (> 1ml) ist es zwingend notwendig, die Sedimentation der Nukleinsäure-Detergenz-Komplexe bei niedrigen g-Zahlen zu erreichen, da ansonsten teure und aufwendige Ultrazentrifugen anstelle einfacher Laborzentrifugen (mit einer maximal erreichbaren g-Zahl von 5000-6000) verwendet werden müßten.

In den Anwendungsbeispielen in US 5,300,635 beschreibt MacFarlane die Zugabe von mindestens 2 Volumen bis hin zu 10 Volumen Detergenz zur Probe. Damit erhöht sich das zu verarbeitende Gesamtvolumen z.T. beträchtlich, insbesondere wenn man an die Aufreinigung von Nukleinsäuren aus mehreren Millilitern Probenmaterial (z.B. Plasmapools) denkt. Die Verarbeitung großer Volumina ist aber vor allem im Hinblick auf eine mögliche Automatisierung der Probenvorbereitung auf einem Pipettierroboter ungünstig, da z.B. die Pipettiervolumina begrenzt sind.

Es bestand daher ein Bedarf an einem Verfahren zur Stabilisierung und Isolierung von Nukleinsäuren, das die oben genannten Nachteil des Stands der Technik nicht aufweist.

Insbesondere bestand ein Bedarf an einem Verfahren, bei dem die Nukleinsäuren in einem einzigen Schritt stabilisiert und die die Nukleinsäuren enthaltende Probe lysiert und die Nukleinsäuren aus der gleichen Lösung isoliert werden können. Dies wird z.B. wichtig, wenn Nukleinsäuren aus solchen Proben stabilisiert/isoliert werden sollen, bei denen es nach Entnahme der Probe zur Induktion von Streßgenen und damit zur Synthese neuer m-RNA-Moleküle kommen kann, wodurch das Transkriptmuster der Zellen verändert werden könnte. Insbesondere bestand weiterhin ein Bedarf an einem Verfahren, bei dem die Komplexe bestehend aus Nukleinsäure und kationischen Verbindungen bei niedrigen g-Zahlen sedimentiert werden können. Ferner bestand insbesondere ein Bedarf an einem Verfahren, bei dem nur geringe oder gar keine Mengen Carrier-Nukleinsäuren oder Carrier-Hilfen, z.B. Heparin, notwendig sind. Es bestand außerdem ein Bedarf an einem Verfahren, bei dem geringere Volumina der kationischen Verbindung der Probe zugegeben werden können. Schließlich bestand ein Bedarf an einem Verfahren, das es ermöglicht, bereits nach dem ersten Verfahrensschritt in kleinen Volumina arbeiten zu können.

Diese Aufgaben werden durch das in Anspruch 1 beschriebene Verfahren und die weiteren unabhängigen Ansprüche gelöst. Bevorzugte Ausführungsformen sind in den Unteransprüchen angegeben.

Es soll ein Verfahren zur Stabilisierung und Isolierung von Nukleinsäuren aus einer biologischen Probe geschaffen werden. Diese Aufgabe wird erfindungsgemäß durch das Verfahren zur Stabilisierung und Isolierung von Nukleinsäuren aus einer biologischen Probe gemäß unabhängigen Patentanspruch 1 gelöst.

Erfindungsgemäß wird zur Stabilisierung und Isolierung von Nukleinsäuren eine biologische Probe mit mindestens einer kationischen Verbindung der Formel (I) in Kontakt gebracht.

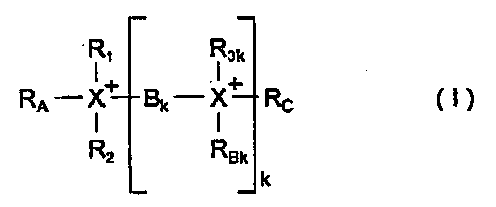

Unter dem Begriff "kationische Verbindung" wird im Rahmen der vorliegenden Erfindung eine Verbindung mit mehr als einer positiven Ladung verstanden. Die als Formel (I) wiedergegebene kationische Verbindung wird in gelöster Form und/oder in Form eines Salzes eingesetzt, wobei die Neutralisierung der Ladung durch konjugierte Basen von starken und/oder schwachen anorganischen und/oder organischen Säuren erfolgt, die im folgenden mit "A" abgekürzt werden. Dies bedeutet, daß das Produkt aus Ladung und Anzahl an Basen A genau die positiven Ladungen der restlichen Verbindung kompensiert.

In der oben genannten Formel (I) steht X für Stickstoffatome (N) oder Phosphoratome (P). In Formel (Ia) ist die kationische Verbindung mit X = N gezeigt, und in Formel (Ib) mit X = P.

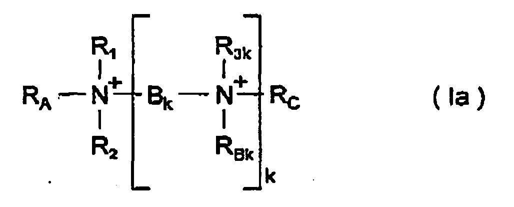

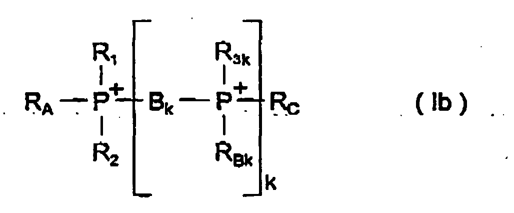

Daneben bedeutet in allen drei vorgenannten Formeln (I), (Ia) und (Ib) k die ganze Zahl 1, 2, 3, 4, 5, 6, 7, 8, 9, 10, 11, 12, 13, 14, 15, 16, 17, 18, 19, 20, 21, 22, 23 oder 24, während Bₖ für aliphatische Alkandiylbrücken steht, in denen ein oder mehrere nicht benachbarte Kohlenstoff-Atome durch Sauerstoff ersetzt sein können und die die Struktur

-(CH₂)ₙ-(OCH₂)ₘ-

aufweisen. Die Alkandiyl-Brücken können im Rahmen der vorliegenden Erfindung an einem oder an mehreren Kohlenstoff-Atomen substituiert sein. Die Parameter n und m sind unabhängig voneinander und bedeuten eine der ganzen Zahl 0, 1, 2, 3, 4, 5 oder 6, wobei n + m > 0 ist.

Alternativ zu den oben angegeben Strukturen bedeutet Bₖ auch eine substituierte Phenyl-, Naphthyl- oder Biphenyl-Brücke der Struktur 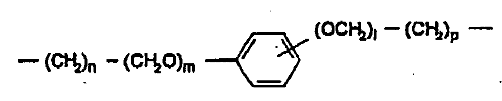 oder 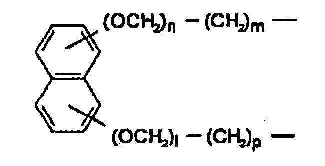 oder 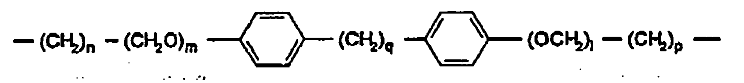 wobei n, m, l, p, q unabhängig voneinander sind und eine der ganzen Zahlen 0, 1, 2, 3, 4, 5 oder 6 bedeuten. Die Phenyl-, Naphthyl- oder Biphenyl-Brücke kann zusätzlich an einem oder an mehreren Kohlenstoff-Atomen substituiert sein.

In der oben angegebenen Formel (I) bedeutet daneben
R₁, R₂, R₃ₖ, die identisch oder unterschiedlich voneinander sein können und die unsubstituiert oder an einem oder mehreren Kohlenstoff-Atomen substituiert sein können, Wasserstoff, ein lineares oder verzweigtes C₁-C₆-Alkyl, ein lineares oder verzweigtes C₁-C₆-Alkenyl, ein lineares oder verzweigtes C₁-C₆-Alkinyl, ein Phenyl, und/oder die gruppen Benzyl, Phenylethyl, Phenylethyl, phenylisopropyl, phenylisobutyl, phenoxymethyl, phenoxyethyl, phenoxypropyl, phenoxyisopropyl, phenoxybutyl 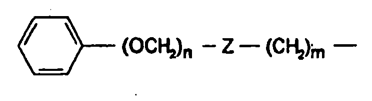 wobei n, m unabhängig voneinander die ganze Zahl 0, 1, 2, 3, 4, 5 oder 6 bedeutet und Z eine der Strukturen -O-, -CO-, - CO₂-, -OCO-, -CO-N-, -N-CO-, -O-CO-N-, -N-CO-O-, -S-oder -S-S-bezeichnet.

Außerdem kann R₁, R₂, R₃ₖ ein Phenyl, ein Benzyl, ein Phenoxyethyl der Struktur 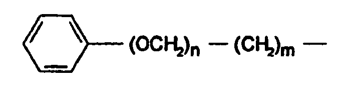 bezeichnen, wobei n, m unabhängig voneinander die ganze Zahl 0, 1, 2, 3, 4, 5 oder 6 bedeutet.

R_{A}, R_{Bk}, R_{C}, die identisch oder unterschiedlich voneinander sein können und die unsubstituiert oder an einem oder mehreren Kohlenstoff-Atomen substituiert sein können, bedeuten Wasserstoff, ein lineares oder verzweigtes C₁-C₂₁-Alkyl, ein lineares oder verzweigtes C₁-C₂₁-Alkenyl, ein lineares oder verzweigtes C₁-C₂₁-Alkinyl, oder eine Struktur

CH₃-(CH₂)ₙ-Z-(CH₂)ₘ-

wobei n die ganze Zahl 0, 1, 2, 3, 4, 5, 6, 7, 8, 9, 10, 11, 12, 13, 14, 15, 16, 17, 18, 19, 20, 21, 22, 23 oder 24 bedeutet, m unabhängig davon die ganze zahe 1, 2, 3, 4, 5, 6, 7, 8, 9, 10, 11, 12, 13, 14, 15, 16, 17, 18, 19, 20, 21, 22, 23 oder 24 bedeutet und Z eine der Strukturen -O-, - CO-, -CO₂-, -OCO-, -CO-N-, -N-CO-, -O-CO-N-, -N-CO-O-, -S-oder -S-S- bezeichnet.

Alternativ bilden R_{A} und R_{C} zusammen einen Rest R_{AC} und somit eine cyclische Struktur 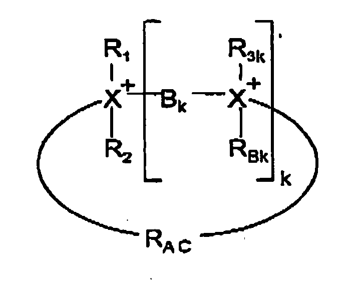 wobei der Rest E_{nc}, der unsubstituiert oder an einem oder mehreren Kohlenstoff-Atomen substituiert sein kann, ein lineares oder verzweigtes C₁-C₈-Alkyl, ein lineares oder verzweigtes C₁-C₈-Alkenyl, oder ein lineares oder verzweigtes C₁-C₈-Alkinyl bedeutet.

Für den Fall, daß k > 1 ist, können die Brückengruppen Bₖ sowie die Gruppen R_{Bk} und R₃ₖ gleich oder verschieden voneinander sein.

Die oben angegebenen Verbindungen werden in dem erfindungsgemäßen Verfahren verwendet, wodurch Nukleinsäuren in einem einzigen Schritt stabilisiert, die die Nukleinsäuren enthaltende Probe lysiert und die Nukleinsäuren isoliert werden können. Die stabilisierten Nukleinsäuren sind nicht nur während der Präparation sondern auch über einen längeren Zeitraum, wie z.B. 96 h oder mehr, stabil. Insbesondere können die Komplexe bestehend aus Nukleinsäure und kationischer Verbindung bei niedrigen g-Zahlen sedimentiert werden, wobei nur geringe oder gar keine Mengen Carrier-Nukleinsäuren oder Carrier-Hilfen notwendig sind. Dabei müssen nur geringe Volumina oder Mengen der kationischen Verbindung der Probe zugegeben werden. Ferner kann durch die Pelletierung der Komplexe bereits nach diesem Schritt in kleinen Volumina gearbeitet werden.

Durch die erfindungsgemäße Stabilisierung von Nukleinsäuren wird erreicht, daß die Nukleinsäuren in einer Probe auch bei längerer Lagerung oder während eines Transports ihre Struktur nicht verändern und die Genauigkeit später durchgeführter Tests wird deutlich erhöht. In manchen Fällen, z.B. wenn Nukleinsäure-haltiges Material über weite Strecken transportiert oder länger gelagert werden muß, macht das erfindungsgemäße Verfahren diese Tests überhaupt erst möglich.

Die Verbindung kann in Lösung oder auch als Feststoff zugesetzt werden. Die Möglichkeit einer Zugabe als Feststoff beinhaltet die weiteren Vorteile, daß Feststoffe meist chemisch stabiler sind und ihre Zugabe zur Probe oft einfacher durchführbar ist. Es kann eine kationische Verbindung oder eine Mischung aus zwei oder mehr kationischen Verbindungen zugefügt werden.

In dem erfindungsgemäßen Verfahren werden bevorzugt Verbindungen der oben angegebenen allgemeinen Formel (I) verwendet, wobei ein Anion A ausgewählt aus der Gruppe: Fluorid, Chlorid, Bromid, Jodid, Perchlorat, Perbromat, Periodat, Phosphat, Hydrogenphosphat, Dihydrogenphosphat, Sulfat, Thiosulfat, Hydroxid, Carbonsäuren, α-Halogen-Carbonsäuren und/oder Hydroxy-Carbonsäuren verwendet wird, und k für die ganze Zahl 1, 2, 3, 4, 5 oder 6 steht, während für den Fall, daß Bₖ eine substituierte Phenyl-, Naphthyl- oder Biphenyl-Brücke bezeichnet, n, m, l, p, q unabhängig voneinander die ganze Zahl 0, 1 oder 2 bedeutet.

In den erfindungsgemäß bevorzugten Verbindungen der allgemeinen Formel (I) bedeuten die Reste R₁, R₂ und R₃ₖ, die identisch oder unterschiedlich voneinander sein können, die C₁-C₆-Alkyl-Gruppen methyl, ethyl, propyl, isopropyl, butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl und/oder 1-ethyl-2-methyl-propyl, und/oder die C₃-C₆-Alkenyl-Gruppen 2-propenyl (allyl), 2-butenyl, 3-butenyl, 1-methyl-2-propenyl, 2-methyl-2-propenyl, 2-pentenyl, 3-pentenyl, 4-pentenyl, 1-methyl-2-butenyl, 2-methyl-3-butenyl, 3-methyl-3-butenyl, 1,1-dimethyl-2-propenyl, 1,2-dimethyl-2-propenyl, 1-ethyl-2-propenyl, 2-hexenyl. 3-hexenyl, 4-hexenyl, 5-hexenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-2-butenyl, 1,2-dimethyl-3-butenyl, 1,3-dimethyl-2-butenyl, 1,3-dimethyl-3-butenyl, 2,2-dimethyl-3-butenyl, 2,3-dimethyl-2-ethyl-2-butenyl, 2-ethyl-3-butenyl, 1,1,2-trimethyl-2-propenyl, 1-ethyl-1-methyl-2-propenyl und/oder 1-ethyl-2-methyl-2-propenyl, und/oder die C₃-C₆-Alkinyl-Gruppen 2-propinyl (propargyl), 2-butinyl, 3-butinyl, 2-pentinyl, 3-pentinyl, 4-pentinyl, 3-methyl-2-butinyl, 2-hexinyl, 3-hexinyl, 4-hexinyl, 5-hexinyl, 3-methyl-2-pentinyl, 4-methyl-2-pentinyl, 2-methyl-3-pentinyl, 4-methyl-3-pentinyl, 1-methyl-4-pentinyl, 1,1-dimethyl-2-butinyl, 1,1-dimethyl-3-butinyl, 1,2-dimethyl-3-butinyl, 1,3-dimethyl-2-butinyl, 2,2-dimethyl-3-butinyl, 1-ethyl-2-butinyl, 1-ethyl-3-butinyl, 2-ethyl-3-butinyl und/oder 1-ethyl-1-methyl-2-propinyl

Die Reste R_{A}, R_{Bk}, R_{C}, die identisch oder unterschiedlich voneinander sein können, bedeuten die linearen oder verzweigten C₈-C₂₀-Alkyl-Gruppen octyl, decyl, undecyl, dodecyl, tridecyl, tetradecyl, pentadecyl, hexadecyl, heptadecyl, octadecyl, nonadecyl und/oder eicosyl und/oder die linearen oder verzweigten C₈-C₂₀-Alkenyl-Gruppen octenyl, decenyl, undecenyl, dodecenyl, tridecenyl, tetradecenyl, pentadecenyl, hexadecenyl, heptadecenyl, octadecenyl, nonadecenyl und/oder eicosenyl und/oder die linearen oder verzweigten C₈-C₂₀-Alkinyl-Gruppen octinyl, decinyl, undecinyl, dodecinyl, tridecinyl, tetradecinyl, pentadecinyl, hexadecinyl, heptadecinyl, octadecinyl, nonadecinyl und/oder eicosinyl und/oder eine Struktur

CH₃-(CH₂)ₙ-Z-(CH₂)ₘ -

wobei n, m unabhängig voneinander sind und n die ganze Zahl 2, 3 oder 4 bedeutet und m die ganze Zahl 8, 9, 10, 11, 12, 13, 14, 15, 16, 17 oder 18 bedeutet und Z eine der Strukturen -O-, -CO-, -OCO-, -CO-N- oder -N-CO- bezeichnet.

Bevorzugt werden im Rahmen der vorliegenden Erfindung Verbindungen der allgemeinen Formel (I) verwendet, wobei eine oder mehrere der als R_{A}, R_{Bk} und R_{C} bezeichneten Gruppen eine der Strukturen

CH₃-(CH₂)ₙ-O-(CH₂)ₘ-

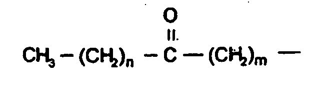

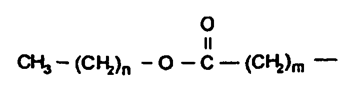

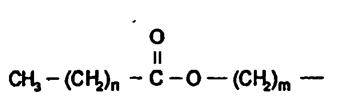

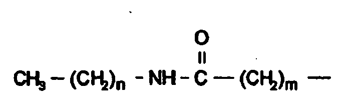

oder 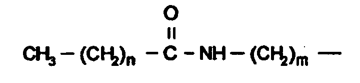 bedeuten.

Unter den oben genannten, erfindungsgemäß bevorzugten Verbindungen werden die Verbindungen ganz besonders bevorzugt, die in einem oder mehreren der Reste R₁, R₂, R₃ₖ, R_{A}, R_{Bk} und R_{C} eine Doppelbindung oder eine Dreifachbindung aufweisen.

Insbesondere werden die Verbindungen bevorzugt, in denen als Reste R₁, R₂ und/oder R₃ₖ die Allyl-Gruppe verwendet wird.

Insbesondere bevorzugt werden im Rahmen der vorliegenden Erfindung Verbindungen der oben angegebenen allgemeinen Formel (I) verwendet, wobei ein Anion A ausgewählt aus der Gruppe: Bromid, Jodid, Perchlorat, Hydrogenphosphat, Sulfat, Acetat, Trifluoracetat, Trichloracetat, Benzoat, Oxalat, Succinat, Phthalat, Citrat, Tartrat, Maleat, Malonat, Fumarat verwendet wird. Weiterhin bedeutet k die ganze Zahl 1 oder 2, während Bₖ die aliphatischen C₂-C₄-Alkandiylbrücken Ethan-1,1-diyl, Ethan-1,2-diyl, Propan-1,1-diyl, Propan-1,2-diyl, Propan-1,3-diyl, Butan-1,1-diyl, Butan-1,2-diyl, Butan-1,3-diyl und/oder Butan-1,4-diyl bezeichnet. R₁, R₂, R₃ₖ bedeuten methyl, ethyl oder hydroxyethyl, während R_{A}, R_{Bk}, R_{C} die linearen C₈-C₂₀-Alkyl-Gruppen octyl, decyl, undecyl, dodecyl, tridecyl, tetradecyl, pentadecyl, hexadecyl, heptadecyl, octadecyl, nonadecyl und/oder eicosyl bedeuten.

Ganz besonders bevorzugt werden in dem erfindungsgemäßen Verfahren Verbindungen der allgemeinen Formel (I) verwendet, wobei die Reste R₁, R₂ und R₃ₖ identisch sind und/oder R_{A}, R_{Bk} und R_{C} identisch sind und/oder für den Fall, daß k > 1 ist, die Brückengruppen Bₖ identisch sind.

In sämtlichen erfindungsgemäß verwendeten Verbindungen können die Kohlenstoff-Atome der Gruppen R₁, R₂, R₃ₖ, R_{A}, R_{Bk} und R_{C} mit einem oder mehreren Halogenatomen, insbesondere einem oder mehreren Fluor-Atomen, und/oder einer oder mehreren primären, sekundären und/oder tertiären Hydroxylgruppen und/oder einer oder mehreren -SH, -NH₂, -NH- und/oder =N-Gruppen substituiert sein, wobei die Substituenten untereinander identisch oder nicht identisch sein können. Dabei werden die Verbindungen bevorzugt, bei denen der Abstand zwischen dem ersten substituierten Kohlenstoff-Atom und dem in der allgemeinen Formel (I) gezeichneten Stickstoff mindestens zwei kovalente Bindungen beträgt. Dies bedeutet also, daß ein oder mehrere der Kohlenstoff-Atome der Gruppen R₁, R₂, R₃ₖ, R_{A}, R_{Bk} und R_{C}, die nicht direkt an eines der Atome X (Stickstoff oder Phosphor) der Verbindung 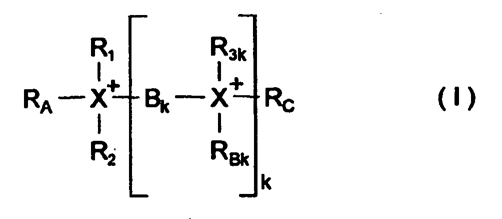 gebunden sind, substituiert sind.

Ebenso können in sämtlichen Ausführungsformen die aliphatischen und/oder aromatischen Kohlenstoff-Atome der Brückgruppen Bₖ mit einem oder mehreren Halogenatomen, insbesondere Fluor-Atomen, und/oder einer oder mehreren primären, sekundären und/oder tertiären Hydroxylgruppen und/oder einer oder mehreren -SH, -NH₂, -NH- und/oder =N-Gruppen und/oder einer oder mehreren linearen oder verzweigten C₁-C₄-Alkylgruppen substituiert sein, wobei die Substituenten untereinander identisch oder nicht identisch sein können. Als Substituenten der Kohlenstoff-Atome der Brückgruppen Bₖ werden insbesondere Methyl-, Ethyl-, Propyl-, i-Propyl-, Butyl-, 2-Methylpropyl- und tert.-Butyl-Gruppen bevorzugt.

Ganz besonders bevorzugt wird das erfindungsgemäße Verfahren mit den kationischen Verbindungen Ethandiyl-1,2-bis(dimethyldecylammoniumbromid), Propandiyl-1,2-bis(dimethyldecylammoniumbromid), Ethandiyl-1,2-bis(dimethyltetradecylammoniumbromid) oder N, N', N"-Tridecyl-N,N,N',N",N"-pentamethyl-bis-(2-ammonioethyl)ammoniumbromid durchgeführt.

Wie bereits angesprochen, kann die mindestens eine kationische Verbindung sowohl als Feststoff, als auch in gelöster Form zu der Probe gegeben werden. Wird die kationische Verbindung in Lösung zugegeben, so werden in dem erfindungsgemäßen Verfahren 0,001 bis 10 Volumen, besonders bevorzugt 0,01-10 Volumen, noch bevorzugter 0,05 bis 2 Volumen und insbesondere bevorzugt 1 Volumen der Lösung der Probe zugegeben, also deutlich geringere Mengen als aus dem Stand der Technik bekannt. Größere oder kleinere Volumina sind auch möglich, falls dies praktische Vorteile bietet. Die Lösung der kationischen Verbindung weist dabei eine Konzentration von 0,01% bis Sättigung, bevorzugt 0,5 bis 5%, besonders bevorzugt 2 bis 4% auf.

Vor dem Inkontaktbringen kann die biologische Probe selbstverständlich vorgereinigt werden, falls dies für die weitere Verarbeitung von Vorteil ist.

Nach dem in Kontakt bringen der kationischen Verbindung mit einer biologischen Probe kann die kationischen Verbindung mit der biologischen Probe vermischt und die Mischung inkubiert werden, wobei bevorzugt 10 Minuten bei Raumtemperatur inkubiert wird.

Gemäß einer bevorzugten Ausführungsform der vorliegenden Erfindung können der kationischen Verbindung und/oder dem gebildeten Komplex aus Nukleinsäure und kationischer Verbindung weitere Mittel zur Unterstützung der Lyse zugefügt werden. Als Mittel zur Unterstützung der Lyse können Alkohole, insbesondere verzweigte oder unverzweigte C1- bis C4-Alkanole wie Isopropanol, Aldehyde, insbesondere niedere C1- bis C4-Aldehyde, verzweigt oder unverzweigt, wie z.B. Glyoxal, Phenole, Phenolderivate, wie z.B. 2-Biphenylol, ionische, zwitterionische und nichtionische Verbindungen, Sulfhydryl reduzierende Reagenzien, insbesondere Dithiothreitol, Phosphorsäurederivate, insbesondere Tributylphosphat, chaotrophe Reagenzien, wie Harnstoff, Carbonsäuren, wie z.B. Zitronensäure oder Malonsäure, oder einfache Salze, wie Ammoniumsalze oder Alkaliphosphate, einzeln oder in Kombination verwendet werden.

Ferner ist es gemäß einer weiteren bevorzugten Ausführungsform der vorliegenden Erfindung auch denkbar, die biologische Probe zu homogenisieren oder mechanisch oder enzymatisch auf sie einzuwirken, bevor oder während die kationische Verbindung zugegeben wurde/wird. Eine mechanische Einwirkung könnte zum Beispiel mit einem elektrischen Messer, einer Kugelmühle, durch Zugabe von Partikeln oder durch Pressen durch eine Spritze geschehen, während geeignete Enzyme zur Einwirkung auf die Probe beispielsweise Hydrolasen, Proteasen oder Lipasen sein könnten. Weitere Möglichkeiten sind dem Fachmann bekannt und sollen hier umfaßt sein. Eine solche Behandlung der biologischen Probe könnte darin vorteilhaft sein, daß die kationische Verbindung besser mit ihren Angriffspunkten in Kontakt treten kann. Erfindungsgemäß werden die gebildeten Komplexe aus Nukleinsäure und kationischer Verbindung durch Zentrifugation sedimentiert. Die Zentrifugation wird bevorzugt bei niedrigen g-Zahlen, insbesondere 500 bis 5000 x g, für 3 bis 10 Minuten durchgeführt. Durch die Sedimentierung der Komplexe in ein kleines Pellet kann die weitere Aufreinigung der Nukleinsäuren in relativ kleinen Volumina durchgeführt werden. Dies ist besonders für Routineanwendungen von Vorteil, im besonderen bei automatisierten Verfahren. Durch die Zentrifugation bei niedrigen g-Zahlen können einfache Laborzentrifugen Verwendung finden.

Die Komplexe können anschließend gegebenenfalls mit einem geeigneten Puffer oder mit Wasser gewaschen werden, wobei Verunreinigungen entfernt werden könnten. Die Komplexe, bestehend aus kationischer Verbindung und Nukleinsäuren, werden anschließend in einem relativ kleinen Volumen eines geeigneten Puffers wieder aufgelöst, wodurch die Nukleinsäuren in dem Puffer freigesetzt werden. Danach können die Nukleinsäuren bei Bedarf mit verschiedenen, bekannten Verfahren in relativ kleinen Volumina weiter aufgereinigt werden. So können sie z.B. nach Einstellung entsprechender Bindebedingungen an eine Membran zur weiteren Aufreinigung gebunden werden. Alternativ zur Abtrennung durch Zentrifugation können die Komplexe aus Nukleinsäure und kationischer Verbindung durch Vakuum, Überdruck, Zentrifugation oder Kapillarkräfte auf einer Oberfläche, z.B. der Oberfläche einer Membran oder auf dem Boden eines Gefäßes konzentriert werden. Gegebenenfalls können die Komplexe hiernach in einer geeigneten Waschlösung gewaschen werden, wodurch vorteilhaft verbleibende Verunreinigungen entfernt werden könnten. Die Komplexe können anschließend durch Zugabe einer geeigneten Reagenzienlösung, gegebenenfalls einschließlich eines Enzyms und/oder mechanischer Einwirkung, unter nicht bindenden oder bindenden Bedingungen aufgelöst werden, wobei die Nukleinsäuren in die Lösung freigesetzt werden. Werden sie unter bindenden Bedingungen aufgelöst, können die Nukleinsäuren z.B. auf derselben Membran wie oben mittels Zentrifugation, Vakuum, Überdruck oder Kapillarkräften gebunden werden, wobei derartige Verfahren z.B. in der PCT-Anmeldung Nr. PCT/EP98/06756 beschrieben sind und hier durch Inbezugnahme inkorporiert sein sollen, und weiter aufgereinigt werden. Werden die Komplexe unter nicht bindenden Bedingungen gelöst, können die freigesetzten Nukleinsäuren mittels Zentrifugation, Vakuum oder Überdruck in einem Sammelröhrchen aufgefangen werden. Sie können anschließend bei Bedarf mit verschiedenen, bekannten Verfahren in relativ kleinen Volumina weiter aufgereinigt werden. So können sie z.B. selbstverständlich nach Einstellung entsprechender Bindebedingungen ebenfalls wieder an eine Membran oder andere Oberfläche zur weiteren Aufreinigung gebunden werden.

Als biologische Probe mit Nukleinsäuren können zellfreies Probenmaterial, Lebensmittelproben, die freie oder gebundene Nukleinsäuren oder Nukleinsäurehaltige Zellen enthalten, Umweltproben, die freie oder gebundene Nukleinsäuren oder Nukleinsäurehaltige Zellen enthalten, eine Suspension von Zellen, Bakterien, Viren oder Hefen, ein Gewebe jeder Art oder eine klinische Probe, wie Blut, Plasma, Serum, Leukozytenfraktionen, Crusta Phlogistica, Sputum, Urin, Sperma, Faeces oder Abstriche, sowie eine Pflanze oder ein Pflanzenteil oder freie Nukleinsäuren verwendet werden, sowie jede weitere denkbare Probe, die Nukleinsäuren enthält.

Die oben spezifizierten kationischen Verbindungen können in einem Kit zur Stabilisierung und Isolierung von Nukleinsäuren verwendet werden das bevorzugt zusätzlich geeignete Puffer enthält. Das Kit kann daneben auch geeignete Mittel zur Unterstützung der Lyse und/oder Mittel zur Aufreinigung der Nukleinsäuren und/oder Mittel zur mechanischen Einwirkung und/oder Mittel zur enzymatischen Behandlung der Probe und/oder der Komplexe enthalten.

Erfindungsgemäß werden die oben spezifizierten kationischen Verbindungen zur Stabilisierung und Isolierung von Nukleinsäuren verwendet. Dabei entsteht ein Komplex, der aus einer Nukleinsäure und einer kationischen Verbindung besteht. Dieser Komplex zeichnet sich durch seine besonders vorteilhafte, hohe Stabilität aus, wodurch Nukleinsäuren vor einem Abbau aus der Probe selbst heraus oder durch Einwirkungen aus der Umgebung geschützt werden.

Die oben spezifizierten kationische Verbindungen oder Komplexe können Verwendung enden in pharmazeutischen Zusammensetzungen, diagnostischen Zusammensetzungen, wobei diese diagnostischen Zusammensetzungen sowohl die Diagnostik im medizinisch-pharmazeutischen Bereich umfassen sollen als auch die Untersuchung von Lebensmittel- und Umweltproben, sowie Zusammensetzungen für die Forschung. Zum Beispiel könnte der entstandene stabilisierte Komplex aus Nukleinsäure und kationischer Verbindung vorteilhaft für die Einschleusung von pharmazeutisch wirksamer NS in erkrankte Zellen genutzt werden.

Das erfindungsgemäße Verfahren kann in einfacher Weise zur Automatisierung der Stabilisierung und Isolierung von Nukleinsäuren herangezogen werden. Die Vorteile des erfindungsgemäßen Verfahrens, nämlich Stabilisierung der Nukleinsäuren, Lyse der die Nukleinsäuren enthaltenden Probe in einem einzigen Schritt und Isolierung der Nukleinsäuren aus der gleichen Lösung, Sedimentierung der Komplexe bestehend aus Nukleinsäure und kationischer Verbindung bei niedrigen g-Zahlen, Verwendung von geringen Mengen an Carrier-Nukleinsäuren oder Carrier-Hilfen oder gar keiner Carrier-Nukleinsäuren oder Carrier-Hilfen, sowie geringer Volumina der kationischen Verbindung sowie geringer Probenvolumina nach der Pelletierung, tragen einzeln und umso mehr in Kombination miteinander zur vereinfachten Automatisierung bei. Die Durchführung in z.B. einem Multi-Well-Modul, wie beispielsweise einem 8-Well oder 96-Well Modul ist ebenfalls möglich.

Die vorliegende Erfindung soll im Rahmen der folgenden Ausführungsbeispiele näher erläutert werden.

Lineare, verzweigte und cyclische kationische Verbindungen werden gemäß den Beispielen 1 oder 2 hergestellt. Zur Bindung der Reste R_{A} und R_{Bk} (für k=1 wird R_{C} anstelle von R_{Bk} verwendet) durch nukleophile Substitution an die Stickstoff-Atome wurden tertiäre Diamine oder tertiäre Polyamine (k>1) mit einer vorbestimmten Zahl an tertiären Stickstoff-Atomen in Lösung mit einem Überschuß an Alkylhalogenid unter Argon-Schutzgas versetzt. Die Stickstoff-Atome sind dabei durch lineare (unverzweigte) Alkandiyl-Brücken oder substituierte Xylylen-Brücken der entsprechenden Länge n miteinander verbunden. Diese an sich bekannte Reaktion zur Quarternierung wurde bei erhöhten Temperaturen durchgeführt. Alkylhalogenide, wie z.B. Alkylbromid oder Alkyliodid wurden im Überschuß verwendet, um zum größten Teil vollständig quarternierte Ammonium-Salze herzustellen. Die so erhaltenen Ammonium-Verbindungen wurden durch Rekristallisation aus verschiedenen Lösungsmitteln und Lösungsmittelgemischen, wie zum Beispiel Diethylether/Methanol, gereinigt.

Alternativ wurden kationische Verbindungen mit zwei kationischen Stickstoff-Atomen (k=1) synthetisiert. Dabei wurden primäre α,ω̅-Alkanyldihalogenide unter den Reaktionsbedingungen gemäß Beispiel 1 mit einem Überschuß an Alkyldimethylamin umgesetzt. Die Alkyl-Kette der Amin-Verbindung kann hydroxyliert sein, weist aber keine HalogenAtome auf. Die Reinigung der kationischen Verbindungen erfolgt wie oben beschrieben.

Ein Austausch der Gegenionen (Anionen A) kann mit Hilfe einer Ionenaustauschersäule erfolgen. Exemplarisch ist in Beispiel 3 der Austausch von Bromid gegen Acetat beschrieben.

### Beispiel 1: Synthese von Ethandiyl-1,2-bis(dimethyl-decyl-ammoniumbromid)

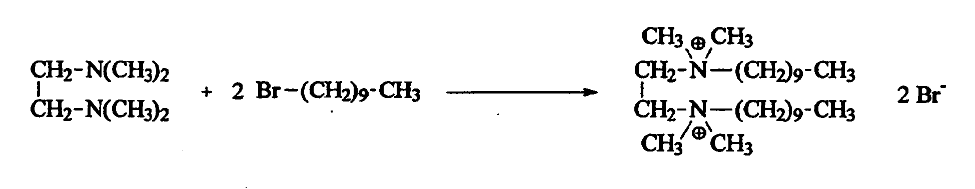

In einem 21 Rundkolben mit Rückflußkühler, Heizpilz und Magnetrührer wurde eine Lösung von 46,0 ml N,N,N',N'-Tetramethylethylendiamin (35,4 g, 0,30 mol) und 151,4 ml 1-Bromdekan (161,8 g, 0,73 mol, 20% Überschuß) in 850 ml Acetonitril und 280 ml Aceton für 42 h auf Rückflußtemperatur erhitzt. Anschließend wurde das Reaktionsgemisch auf Raumtemperatur abgekühlt und dann eisgekühlt um die Kristallisation der Reaktionsprodukte zu vervollständigen. Die Kristallmasse wurde dann abgenutscht und zweimal mit insgesamt 200 ml kaltem Aceton gewaschen. Das feste Reaktionsprodukt wurde dann in einen 21 Rundkolben mit Rückflußkühler überführt und mit 1,8 l Diethylether versetzt. Nachdem die Rückflußtemperatur erreicht war wurden bis zur vollständigen Auflösung des Feststoffs kleine Mengen Methanol zugegeben. Insgesamt wurden dabei rund 350 ml Methanol zugegeben. Das Produkt kristallisierte über Nacht bei 4 °C aus und wurde dann abgenutscht und im Vakuumtrockenschrank bei 60 °C getrocknet. Die erste Fraktion ergaben 102 g trockenes Produkt (60% theoret. Ausbeute). Eine zweite Fraktion aus dem Reaktionsansatz ergaben nach dem Umkristallisieren 1,8 g trockenes Produkt. Die DC-Analyse des trockenen Produkts (Silica RP18 Platte; mobile Phase: Chloroform 25%, Methanol 16%, n-Propanol 25%, Ethylacetat 25%, 0,25%ige wässrige Kaliumchlorid-Lösung 9%) ergab nach Anfärbung in der Iodkammer einen neuen Substanzfleck. Edukte waren keine mehr vorhanden.

Analog zu der oben beschriebenen Darstellung von Ethandiyl-1,2-bis(dimethyl-decyl-ammoniumbromid) erfolgt die Darstellung der cyclischen Verbindungen, also der Verbindungen, in denen die Reste R_{A} und R_{C} zusammen einen Rest R_{AC} bilden. Exemplarisch sei hier die Reaktionsgleichung der Darstellung von N,N'-Dioctadecyl-N,N'-dimethylpiperazindiiumdibromid aus 1,4-Dimethylpiperazin und Octadecylbromid angegeben:

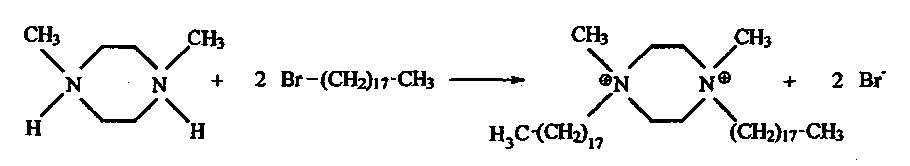

### Beispiel 2: Synthese von N,N',N"-Tritetradecyl-N,N,N',N'',N''-pentamethyl-bis-(2-ammonioethyl)-ammoniumbromid

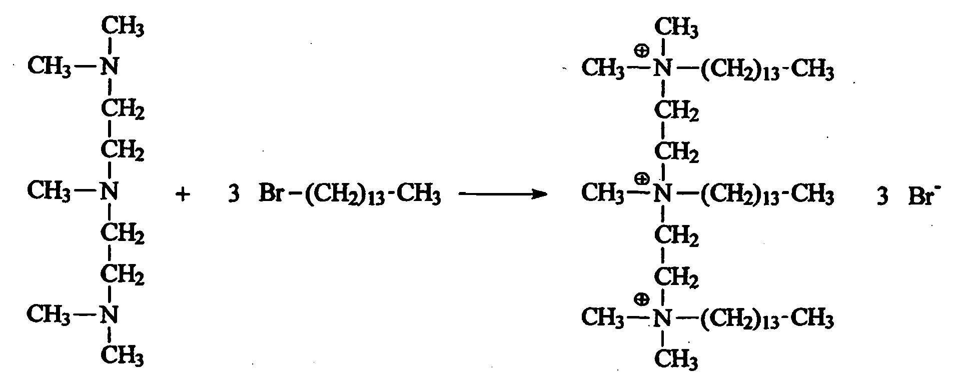

In einem 21 Rundkolben mit Rückflußkühler, Heizpilz und Magnetrührer wurde eine Lösung von 20,9 ml N,N,N',N',N"-pentamethyldiethylentriamin (17,3 g, 0,10 mol) und 93,5 ml 1-Bromtetradecan (99,8 g, 0,36 mol, 20% Überschuß) in 500 ml Acetonitril und 150 ml Aceton über 72 h auf Rückflußtemperatur erhitzt.

Anschließend wurde das Reaktionsgemisch auf Raumtemperatur abgekühlt und über Nacht bei 4°C gelagert um die Kristallisation der Reaktionsprodukte zu vervollständigen. Das kristallisierte Feststoff wurde abgenutscht und zweimal mit insgesamt 200 ml kaltem Aceton gewaschen. Der Feststoff wurde in einen 11 Rundkolben mit Rückflußkühler überführt, mit 1,81 Diethylether versetzt und auf Rückfluß erhitzt. Um den Feststoff komplett zu lösen wurde 250ml Methanol zugegeben. Die Lösung wurde dann auf Raumtemperatur abgekühlt und über Nacht bei 4°C gelagert. Das ausgefallene Produkt wurde abfiltriert und bei 60°C im Vakuumtrockenschrank getrocknet. Die Ausbeute betrug 60,1 g (59% der theoret. Ausbeute). Aus dem Filtrat des ursprünglichen Reaktionsansatzes konnten nach Umkristallisieren weitere 3,1 g Produkt erhalten werden. Die DC-Analyse (Silica RP18 Dünnschichtplatte; mobile Phase: Chloroform 25%, Methanol 16%, n-Propanol 25%, Ethylacetat 25%, 0,25%ige wäßrige Kaliumchlorid-Lösung 9%) zeigt nach Anfärben in der Iodkammer einen neuen Spot. Edukte konnten nicht mehr nachgewiesen werden.

### Beispiel 3: Darstellung von kationischen Verbindungen mit Acetat als Gegenanion

Eine chromatographische Säule wurde mit 8g Dowex^{®} 1x8-400 Anionentauscher gepackt. Die Säule wurde gründlich mit 50% wässrigem Methanol gewaschen bis das Eluat farblos war. Die Säule wurde dann mit insgesamt 20 Säulenvolumen einer 1M Essigsäure beladen, mit destilliertem Wasser bis zur Neutralität gewaschen und schließlich mit 10 Säulenvolumen 50 % wässrigem Methanol gespült. Nach diesen Waschschritten wurde eine Lösung von 1 g der kationischen Verbindung in der Bromidform in 2 ml 50 % wässrigem Methanol mit einer Flußrate von 1 ml/min auf die Säule gegeben. Die Verbindung wurde in 15 Säulenvolumen mit 50% wässrigem Methanol eluiert. Durch Gefriertrocknung wurde das Produkt aus dem Eluat isoliert.

Gemäß den als Beispiel 1 angegebenen Reaktionen wurden die folgenden Verbindungen, die alle aus Diethylether/Methanol umkristallisiert wurden, hergestellt:

| **Edukte** | | **Reaktionszeit** | **Ausbeute** | **Produkt** |
|---|---|---|---|---|
| Tetramethylethyliamin | Octyibromid | 42 h | 45% | Ethandiyl-1,2-bis(octyl dimethyl ammonium bromid) |
| Tetramethylethyliamin | Decylbromid | 42 h | 65% | Ethandiyl-1,2-bis(decyl dimethyl ammonium bromid) |
| Tetramethylethylendiamin | Dodecylbromid | 42 h | 63% | Ethandiyl-1,2-bis(dodecyl dimethyl ammonium bromid) |
| Tetramethylethylendiamin | Tetradecylbromid | 42 h | 35% | Ethandiyl-1,2-bis(tetradecyl dimethyl |
| Tetramethylethylendiamin | Hexadecylbromid | 42 h | 41% | ammonium bromid) Ethandiyl-1,2-bis(hexadecyl dimethyl ammonium bromid) |
| Tetramethylethylendiamin | Octadecylbromid | 42 h | 14% | Ethandiyl-1,2-bis(octadecyl dimethyl ammonium bromid) |
| 1,4-Dimethylbiperazin | Octadecylbromid | 42 h | 42% | N,N'-Dioctadecyl-N,N'-dimethyl piperazin-diiumdibromid |
| Tetramethylpropandiamin | Decylbromid | 42 h | 77% | Propandiyl-1,3bis(decyl dimethyl ammonium bromid) |
| Tetramethylpropandiamin | Dodecylbromid | 42 h | 85% | Propandiyl-1,3-bis(dodecyl dimethyl ammonium bromid) |
| Tetramethylpropandiamin | Tetradecylbromid | 42 h | 55% | Propandiyl-1,3-bis(tetradecyl dimethyl ammonium bromid) |
| Tetramethylpropandiamin | Hexadecylbromid | 42 h | 91% | Propandiyl-1,3-bis(hexadecyl dimethyl ammonium bromid) |
| Tetramethylpropandiamin | Octadecylbromid | 42 h | 87% | Propandiyl-1,3-bis(octadecyl dimethyl ammonium bromid) |
| Tetramethylbutandiamin | 1-Brom-3-methylbutan | 42h | 98% | Ethandiyl-1,3-bis(3-methylbutyl dimethyl ammonium bromid) |
| Tetramethylbutandiamin | Decylbromid | 42 h | 78% | Butandiyl-1,4-bis(decyl dimethyl ammonium bromid) |
| Tetramethylbutandiamin | Dodecylbromid | 42 h | 82% | Butandiyl-1,4-bis(dodecyl dimethyl ammonium bromid) |
| Tetramethylbutandiamin | Tetradecylbromid | 42 h | 58% | Butandiyl-1,4-bis(tetradecyl dimethyl ammonium bromid) |
| Tetramethylbutandiamin | Hexadecylbromid | 42 h | 50% | Butandiyl-1,4-bis(hexadecyl dimethyl ammonium bromid) |
| Tetramethylbutandiamin | Octadecylbromid | 42 h | 32% | Butandiyl-1,4-bis(aoctadecyl dimethyl ammonium bromid) |
| Tetramethylbutandiamin | Eicosylbromid | 42h | 72% | Butandiyl-1,4-bis(eicosyl dimethyl ammonium bromid) |
| Pentamethyldiethylentriamin | Octylbromid | 42 h | 13% | N,N',N"-trioctyl-N,N,N',N",N"-pentamethyl-bis-(2-ammonioethyl)-ammoniumbromid |
| Pentamethyldiethylentriamin | Decylbromid | 42 h | 53% | N,N',N"-tridecyl-N,N,N',N",N"-pentamentyl-bis-(2-ammonioethyl)-ammoniumbromid |
| Pentamethyldiethylentriamin | Dodecylbromid | 42 h | 42% | N,N',N"-tridodecyl-N,N,N',N",N"-pentamethyl-bis-(2-ammonioethyl)-ammoniumbromid |
| Pentamethyldiethylentriamin | Tetradecylbromid | 42 h | 54% | N,N',N"-tritetradecyl-N,N,N',N",N"-pentamethyl-bis-(2-ammonioethyl)-ammoniumbromid |
| Pentamethyldiethylentriamin | Hexadecylbromid | 42 h | 58% | N,N',N"-trihexadecyl-N,N,N',N",N"-pentamethyl-bis-(2-ammonioethyl)-ammoniumbromid |
| Pentamethyldiethylentriamin | Octadecylbromid | 42 h | 30% | N,N',N"-trioctadecyl-N,N,N",N"-pentamethyl-bis-(2-ammonioethyl)-ammoniumbromid |
| Pentamethyldiethylentriamin | Eicosylbromid | 42 h | 38% | N,N',N"-trieiosyl-N,N',N",N"-pentamethyl bis-(2-ammonioethyl)-ammoniumbromid |

Gemäß der angegebenen Alternative wurden die folgenden Verbindungen, die alle aus Diethylether/Methanol umkristallisiert wurden, hergestellt:

| **Edukte** | | **Reaktionszeit** | **Ausbeute** | **Produkt** |
|---|---|---|---|---|
| 1,2-Dibromethan | Decylamin | 48h | 53% | Ethandiyl-1,2-bis(decyl dimethyl ammonium bromid) |
| 1,2-Dibromethan | Dodecylamin | 48h | 55% | Ethandiyl-1,2-bis(dodecyl dimethyl ammonium bromid) |
| 1,2-Dibromethan | Tetradecytamin | 48h | 53% | Ethandiyl-1,2-bis(tetradecyl imethyl ammonium bromid) |
| 1,2-Dibromethan | Hexadecylamin | 48h | 50% | Ethandiyl-1,2-bis(hexadecyl dimethyl ammonium bromid) |
| 1,2-Dibromethan | Octadecytamin | 48h | 48% | Ethandiyl-1,2-bis(octadecyi dimethyl ammonium bromid) |
| 1,3-Dibrompropan | Decylamin | 48h | 68% | Propandiyl-1,3-(decyl dimethyl ammonium bromid) |
| 1,3-Dibrompropan | Dodecylamin | 48h | 65% | Propandiyl-1,3-bis(dodecyl dimethyl ammonium bromid) |
| 1,3-Dibramprapan | Tetradecytamin | 48h | 63% | Propandiyl-1,3bis(tetrdecyl dimethyl ammonium bromid) |
| 1,3-Dibrompropan | Hexadecylamin | 48h | 64% | Proandiyl-1,3-bis(hexadecyl dimethyl ammonium bromid) |
| 1,3-Dibrompropan | Octadecylamin | 48h | 60% | Propandiyl-1,3-bis(octadecyl dimethyl ammonium bromid) |
| 1,4-Dibrombutan | Decylamin | 48h | 65% | Butandiyl-1,4-b-bis(decyl dimethyl ammonium bromid) |
| 1,4-Dibrombutan | Dodecylamin | 48h | 66% | Butandiyl-1,4-bis(dodecyl dimethyl ammonium bromid) |
| 1,4-Dibrombutan | Tetradecytamin | 48h | 63% | Butandiyl-1,4-bis(tetradecyl dimelhyl ammonium bromid) |
| 1,4-Dibrombutan | Hexadecytamin | 48h | 65% | Butandiyl-1,4-bis(hexadecyl dimethyl ammonium bromid) |
| 1,4-Dibrombutan | Octadecylamin | 48h | 60% | Butandiyl-1,4-bis(octadecyl dimethyl ammonium bromid) |

### Beispiel 4: Referenzbeispiel

Als Modell für die Isolierung von Virus RNA aus Plasma dient ein radioaktiv markiertes, 4,5 kB langes in vitro Transkript des Evx-Genes aus der Maus. Die radioaktive Markierung erfolgt dabei durch den Einbau von α³²P-UTP durch die T7-RNA-Polymerase in das RNA-Transkript.

### Versuch A

In einem 1,5 ml Reaktionsgefäß werden zu 140 µl Plasma 4 Volumen (560 µl) einer 3,6 %igen Lösung von Tetradecyltrimethylammonium Oxalat gegeben. In den Deckel des Reaktionsgefäßes wird Carrier RNA (poly A RNA einer Länge von 700 Basen bis zu 7 kB) in unterschiedlicher Menge gegeben, sowie radioaktiv markiertes Transkript. Der Deckel des Reaktionsgefäßes wird geschlossen, die Probe gut gemischt und für 10 min bei Raumtemperatur inkubiert. Die Komplexe bestehend aus RNA und kationischer Verbindung werden für 2 min bei 10000 x g sedimentiert, der Überstand abgenommen und das Pellet in 600 µl eines Guanidiniumthiocyanat haltigen Puffers resuspendiert und mit 1 Volumen 70 %igem Ethanol versetzt. Die Probe wird auf eine Silica-Membran enthaltende spin-Säule aufgetragen und mittels Zentrifugation für 1 min bei ca. 6000 x g durch die Membran hindurchgeführt. Die spin-Säule wird zweimal mit einem Ethanol und NaCl haltigen Puffer gewaschen, wobei der Puffer wiederum durch Zentrifugation für 1 min bei ca. 6000 x g durch die Membran geführt wird. Die Membran wird für 3 min bei 20 000 x g trockenzentrifugiert und die RNA mit 50 µl RNase freiem Wasser mittels Zentrifugation für 1 min bei ca. 10 000 x g eluiert.

Während des Prozesses werden alle Fraktionen (Überstand, Durchbruch, Waschpuffer, spin-Säule und Eluat) gesammelt und anschließend wird die Verteilung des radioaktiv markierten Transkriptes in den einzelnen Fraktionen durch Messung in einem Szintillationszähler bestimmt.

**Tabelle 1: Verteilung der radioaktiv markierten RNA im Überstand und im Eluat in Abhängigkeit von der Carriermenge. Die Differenz zu 100 % ergibt sich aus den Anteilen an RNA in den übrigen Fraktionen (Spin-Säule und Waschpuffer).**

| Carriermenge / µg | RNA im Überstand / % | RNA im Eluat / % |
|---|---|---|
| 0 | 83 | 3 |
| 2,5 | 79 | 13 |
| 5 | 63 | 26 |
| 7,5 | 49 | 39 |
| 10 | 30 | 55 |
| 25 | 8 | 75 |

### Versuch B

In einem 15 ml Reaktionsgefäß wird zu 1 ml Plasma 2 ml einer 3,6 %igen Lösung von Tetradecyltrimethylammonium Oxalat gegeben. In den Deckel des Reaktionsgefäßes wird Carrier RNA (poly A RNA einer Länge von 700 Basen bis zu 7 kB) in unterschiedlicher Menge gegeben, sowie radioaktiv markiertes Transkript. Der Deckel des Reaktionsgefäßes wird geschlossen, die Probe gut gemischt und für 10 min bei Raumtemperatur inkubiert. Die Komplexe bestehend aus RNA und kationischer Verbindung werden für 10 bis 40 min bei ca. 4500 x g sedimentiert.

Anschließend wird der Anteil des radioaktiv markierten Transkriptes im Sediment und im Überstand durch Messung in einem Szintillationszähler bestimmt.

**Tabelle 2: Anteil radioaktiv markierter RNA (in %) im Sediment in Abhängigkeit von der Carriermenge sowie der Zentrifugationszeit. Die Differenz zu 100 % ergibt sich aus dem Anteil an RNA im Überstand.**

| Zentrifugationszeit / min | 50 µg Carrier | 100 µg Carrier | 150 µg Carrier |
|---|---|---|---|
| 10 | 22 % | nd | nd |
| 20 | 31 % | 79 % | 84 % |
| 30 | 36 % | nd | nd |
| 40 | 46 % | 89 % | 91 % |

Die beiden Versuche zeigen, daß sowohl sehr hohe Cariermengen als auch hohe g-Zahlen nötig sind, um die RNA/Tetradecyltrimethylammonium Oxalat-Komplexe zu pelletieren.

### Beispiel 5:

Die Vorteile des erfindungsgemäßen Verfahrens werden in den folgenden Beispielen dargestellt.

Als Modell für die Isolierung von Virus RNA aus Plasma dient ein radioaktiv markiertes 4,5 kB langes in vitro Transkript des Evx-Genes aus der Maus. Die radioaktive Markierung erfolgt dabei durch den Einbau von alpha ³²P-UTP durch die T7-RNA-Polymerase in das RNA-Transkript.

In einem 15 ml Reaktionsgefäß werden zu 1 ml Plasma 1 ml einer 0,5 %igen Lösung von Ethandiyl-1,2-bis(dimethyldecylammoniumbromid) gegeben. In den Deckel des Reaktionsgefäßes wird Carrier RNA (poly A RNA einer Länge von 700 Basen bis zu 7 kB) in unterschiedlicher Menge gegeben, sowie radioaktiv markiertes Transkript. Der Deckel des Reaktionsgefäßes wird geschlossen, die Probe gut gemischt und für 10 min bei Raumtemperatur inkubiert. Die Komplexe bestehend aus RNA und kationischer Verbindung werden für 20 min bei ca. 4500 x g sedimentiert.

Anschließend wird der Anteil des radioaktiv markierten Transkriptes im Sediment und im Überstand durch Messung in einem Szintillationszähler bestimmt.

Tabelle 3: Anteil radioaktiv markierter RNA (in %) im Sediment in Abhängigkeit von der Carriermenge sowie der Zentrifugationszeit. Die Differenz zu 100 % ergibt sich aus dem Anteil an RNA im Überstand.

| Carriermenge / µg | RNA im Sediment / % |
|---|---|
| 0 | 95 % |
| 5 | 96 % |
| 10 | 94 % |

Trotz geringer oder gar keiner Carriermengen und einer Sedimentation der Komplexe bestehend aus RNA und kationischer Verbindung bei niedrigen g-Zahlen wird eine hohe RNA-Ausbeute im Sediment erhalten.

### Beispiel 6: Konzentrierung der Komplexe bestehend aus RNA und kationischer Verbindung auf unterschiedlichen Membranen

Es wird 200 µl Plasma mit 200 µl einer 1 %igen Lösung von Ethandiyl-1,2-bis(dimethyldecylammoniumbromid) gemischt. In den Deckel des Reaktionsgefäßes wird radioaktiv markiertes Transkript gegeben (s. Beispiel 5). Es wird keine zusätzliche Carrier-RNA zugesetzt. Der Deckel des Reaktionsgefäßes wird geschlossen, die Probe gut gemischt und für 10 min bei Raumtemperatur inkubiert. Die Komplexe bestehend aus RNA und kationischer Verbindung werden auf unterschiedlichen Membranen konzentriert, indem sie durch Zentrifugation für 2 min bei 10000 x g in spin-Säulen, die eine entsprechende Membran enthalten, die zur mechanischen Unterstützung auf einer Polypropylenfritte plaziert wird und mit einem Spannring fixiert wird, durch diese Membran geführt werden. Die löslichen Bestandteile binden hierbei nicht an der Membran.

Anschließend wird der Anteil des radioaktiv markierten Transkriptes im Durchbruch und auf der spin-Säule durch Messung in einem Szintillationszähler bestimmt.

**Tabelle 4: Anteil radioaktiv markierter RNA (in %), die auf der entsprechenden Membran zurückgehalten wurde. Die Differenz zu 100 % ergibt sich aus dem Anteil an RNA im Durchbruch. Es wurden jeweils Doppelbestimmungen durchgeführt.**

| **Membran** | **Ausbeute** |
|---|---|
| Pall Hydrolon HNPH 3R 3µm Porengröße | 21 % |
| Nylon, hydrophob | 20 % |
| Pall Hydrolon 1,2 µm Porengröße | 39 % |
| Nylon, hydrophob | 38 % |
| Pall (FluoRepel) Supor 450 | 43 % |
| Polyethersulfon, hydrophob | 46 % |
| Pall Fluorotrans PVDF 0,2 µm | 40 % |
| Polyvinylidendifluorid, hydrophob | 40 % |

Das Ergebnis zeigt, daß die Komplexe aus Nukleinsäure und der kationischen Verbindung auch auf geeigneten Membranen aufkonzentriert werden können.

### Beispiel 7: Isolierung von RNA aus Plasma durch Komplexierung mit kationischen Verbindungen und nachfolgender Aufreinigung an einer Silica-Membran

In einem 2 ml Reaktionsgefäß wird zu 1 ml Plasma 1 ml Lysepuffer zugeben, der neben 1 - 20 % (w/v) Ethandiyl-1,2-bis(dimethyldecylammoniumbromid) noch Harnstoff in einer Konzentration von 1 - 6 M, und/oder Tributylphosphat in einer Konzentration von 0,1 - 1 % (v/v), und/oder Dithiothreitol in einer Konzentration von 5 - 40 mM, und/oder Isopropanol in einer Konzentration von 10 - 50 % (w/v) enthalten kann. In den Deckel des Reaktionsgefäßes wird radioaktives Transkript, sowie 10 µg poly A-Carrier-RNA pipettiert (siehe Beispiel 4); der Deckel geschlossen und der Ansatz gut gemischt. Der Ansatz wird 10 min bei Raumtemperatur inkubiert. Die Komplexe aus RNA und kationischer Verbindung werden in der Eppendorf Zentrifuge 5417 für 3 min bei 3000 rpm = ca. 1000 x g sedimentiert und der Überstand abpipettiert. Das Pellet wird in 500 µl eines Trishydroxymethylaminomethan (Tris HCl) Puffers mit einem pH-Wert von 6 - 8 und mit hoher Salzkonzentration z.B. 2 - 5 M LiCl 2 - 5 M Natriumacetat, 4 - 6 M Guanidiniumthiocyanat, oder 2 - 6 M Guanidinhydrochlorid (GuHCl) gelöst. Zur besseren Resuspendierung des Pellets kann der Puffer auf 60°C erwärmt werden. Außerdem kann dem Puffer Proteinase K zugegeben (400 µg) und der Ansatz dann für 10 min bei 60°C inkubiert werden. Dann wird 500 µl einer Lösung, die 40 - 98 % (v/v) Ethanol enthält, zugegeben. Eine oder beide Lösungen können darüber hinaus auch ein nicht ionisches, oder zwitterionisches Detergenz, wie z.B. Triton® X-100, Nonidet-P40, TWEEN® 20, CHAPSO oder ZWITTERGENT® 3-12 in einem Konzentrationsbereich von 1 - 20 % enthalten. Die Lösung wird auf eine Silica-Membran enthaltende spin-Säule geladen und durch Zentrifugation für 1 min bei ca. 3700 x g durch die Membran geführt. Die spin-Säule wird zweimal mit jeweils 700 µl eines Ethanol und NaCl haltigen Waschpuffers gewaschen, wobei der Waschpuffer mit Zentrifugation bei 10 000 x g durch die Membran geführt wird. Die spin-Säule wird für 3 min bei 20 000 x g trockenzentrifugiert, und die RNA in zwei Schritten mit je 30 µl Wasser von der Silica-Membran eluiert.

Während des Prozesses werden alle Fraktionen (Überstand, Durchbruch, spin-Säule und Eluat) gesammelt und anschließend wird die Verteilung des radioaktiv markierten Transkriptes in den einzelnen Fraktionen durch Messung in einem Szintillationszähler bestimmt.

In Tabelle 5 sind exemplarisch Ergebnisse von unter den oben genannten Bedingungen durchgeführten Aufreinigungen von radioaktiv markierter RNA aus Plasma dargestellt.

**Tabelle 5: Ausbeute an radioaktiv markierter RNA im Eluat. Die Angaben erfolgen in Prozent der eingesetzten Gesamtmenge radioaktiver RNA. Die Differenz zu 100 % ergibt sich aus den Anteilen an RNA in den übrigen Fraktionen (Überstand, Durchbruch und Spin-Säule).**

| **Lysepuffer** | **Aufreinigungspuffer** | **Ausbeute** |
|---|---|---|
| 1 % Ethandiyl-1,2- | 2 M GuHCl 50 mM Tris HCl pH 7,5 | 42 % |
| bis(dimethyldecylammoniumbromid), 50 mM Tris HCl pH 7,5 | 80 % (v/v) Ethanol | |
| 1 % Ethandiyl-1,2- | 3 M GuHCl 50 mM Tris HCl pH 7,5 | 45 % |
| bis(dimethyldecylammoniumbromid), 50 mM Tris HCl pH 7,5 | 80 % (v/v) Ethanol | |
| 1 % Ethandiyl-1,2- | 5 M GuHCl 50 mM Tris HCl pH 7,5 | 53 % |
| bis(dimethyldecylammoniumbromid), 50 mM Tris HCl pH 7,5 | 80 % (v/v) Ethanol | |
| 1 % Ethandiyl-1,2- | 6 M GuHCl 50 mM Tris HCl pH 7,0 | 40 % |
| bis(dimethyidecylammoniumbromid), 50 mM Tris HCl pH 7,0 | 40 % (v/v) Ethanol | |
| 1 % Ethandiyl-1,2- | 6 M GuHCl 50 mM Tris HCl pH 7,0 | 32 % |
| bis(dimethyldecylammoniumbromid), 50 mM Tris HCl pH 7,0 | 60 % (v/v) Ethanol | |
| 1 % Ethandiyl-1,2- | 6 M GuHCl 50 mM Tris HCl pH 7.0 | 24 % |
| bis(dimethytdecylammoniumbromid), 50 mM Tris HCl pH 7,0 | 80 % (v/v) Ethanol | |
| 1 % Ethandiyl-1,2- | 6 M GuHCl 50 mM Tris HCl pH 7,0 | 31 % |
| bis(dimethyldecylammoniumbromid), 50 mM Tris HCl pH 7,0 | 98 % (v/v) Ethanol | |
| 20 % Ethandiyl-1,2- | 6 M GuHCl 50 mM Tris HCl pH 7,0 | 50 % |
| bis(dimethyldecylammoniumbromid), 50 mM Tris HCl pH 7,0, 3 M Harnstoff | 80 % (v/v) Ethanol, 10 % (v/v) Nonidet P40 | |
| 20 % Ethandiyl-1,2- | 6 M GuHCl 50 mM Tris HCl pH 7,0 | 32 % |
| bis(dimethyldecylammoniumbromid), | 80 % (v/v) Ethanol, 10 % (v/v) Nonidet P40 | |
| 50 mM Tris HCl pH 7,0, 6 M Harnstoff | | |
| 5 % Ethandiyl-1,2- | 6 M GuHCl 50 mM Tris HCl pH 7,0 | 64 % |
| bis(dimethyldecylammoniumbromid), | 80 % (v/v) Ethanol, 10 % (v/v) Nonidet P40 | |
| 50 mM Tris HCl pH 7,0, 3 M Harnstoff. | | |
| 0,2 % (v/v) Tributylphosphat | | |
| 5 % Ethandiyl-1,2- | 6 M GuHCl 50 mM Tris HCl pH 7,0 | 50 % |
| bis(dimethyldecylammoniumbromid), | 80 % (v/v) Ethanol, 10 % (v/v) Nonidet P40 | |
| 50 mM Tris HCl pH 7,0, 3 M Harnstoff, | | |
| 0,6 % (v/v) Tributylphosphat | | |
| 5 % Ethandiyl-1,2- | 6 M GuHCl 50 mM Tris HCl pH 7,0 | 36 % |
| bis(dimethyldecylammoniumbromid), | 80 % (v/v) Ethanol, 10 % (v/v) Nonidet P40 | |
| 50 mM Tris HCl pH 7,0, 3 M Harnstoff. | | |
| 0,8 % (v/v) Tributylphosphat | | |
| 1 % Ethandiyl-1,2- | 6 M GuHCl 50 mM Tris HCl pH 7,0, | 66 % |
| bis(dimethyldecytammoniumbromid), | 5 % (v/v) Nonidet P40 | |
| 50 mM Tris HCl pH 7,0, 3 M Harnstoff, | 80 % (v/v) Ethanol, 10 % (v/v) Nonidet P40 | |
| 30 % (v/v) Isopropanol | | |
| 1 % Ethandiyl-1,2- | 6 M GuHCl 50 mM Tris HCl pH 7,0, | 49 % |
| bis(dimethyldecylammoniumbromid), | 5 % (v/v) Nonidet P40 | |
| 50 mM Tris HCl pH 7,0, 3 M Harnstoff, | 80 % (v/v) Ethanol, 10 % (v/v) Nonidet P40 | |
| 40 % (v/v) Isopropanol | | |
| 2 % Ethandiyl-1,2- | 6 M GuHCl 50 mM Tris HCl pH 7,0, | 65 % |
| bis(dimethyldecylammoniumbromid), | 5 % (v/v) Nonidet P40 | |
| 50 mM Tris HCl pH 7,0, 3 M Harnstoff, | 80 % (v/v) Ethanol, 10 % (v/v) Nonidet P40 | |
| 30 % (v/v) Isopropanol, 10 mM Dithiotreitol | | |
| 2 % Ethandiyl-1,2- | 6 M GuHCl 50 mM Tris HCl pH 7,0, | 71 % |
| bis(dimethyldecylammoniumbromid), | 5 % (v/v) Nonidet P40 | |
| 50 mM Tris HCl pH 7,0, 4 M Harnstoff, | 80 % (v/v) Ethanol, 10 % (v/v) Nonidet P40 | |
| 30 % (v/v) Isopropanol, 5 mM Dithiotreitol | | |
| 0,3 % (v/v) Tributylphosphat | | |
| 2 % Ethandiyl-1,2- | 6 M GuHCl 50 mM Tris HCl pH 7,0, | 78 % |
| bis(dimethyldecylammoniumbromid), | 1 % (v/v) Nonidet P40, 400 µg Proteinase K | |
| 50 mM Tris HCl pH 7,0, 4 M Harnstoff, | 80 % (v/v) Ethanol, 10 % (v/v) Nonidet P40 | |
| 30 % (v/v) Isopropanol, 5 mM Dithiotreitol | | |
| 0,3 % (v/v) Tributylphosphat | | |
| 1 % Ethandiyl-1,2- | 2 M LiCl 50 mM Tris HCl pH 7,5 | 39 % |
| bis(dimethyldecylammoniumbromid), 50 mM Tris HCl | 80 % (v/v) Ethanol | |
| pH 7,5 | | |
| 1 % Ethandiyl-1,2- | 5 M LiCl 50 mM Tris HCl pH 7,5 | 38 % |
| bis(dimethyldecylammoniumbromid), 50 mM Tris HCl | 80 % (v/v) Ethanol | |
| pH 7,5 | | |
| 2 % Ethandiyl-1,2- | 2 M Natriumacetat pH 6,5 | 31 % |
| bis(dimethyldeoylammoniumbromid), 50 mM Tris HCl | 70 % (v/v) Ethanol | |
| pH 7,5 | | |
| | | |
| 2 % Ethandiyl-1,2- | 4 M Natriumacetat pH 6,5 | 30 % |
| bis(dimethyldecylammoniumbromid), 50 mM Tris HCl | 70 % (v/v) Ethanol | |
| pH 7,5 | | |
| 1 % Ethandiyl-1,2- | 4 M Guanidiniumthiocyanat, 50 mM Tris HCl | 59 % |
| bis(dimethyldecylammoniumbromid), 50 mM Tris HCl | pH 7,0 | |
| pH 7,5 | 80 % (v/v) Ethanol | |
| 1 % Ethandiyl-1,2- | 6 M Guanidiniumthiocyanat, 50 mM Tris HCl | 46 % |
| bis(dimethyldecylammoniumbromid), 50 mM Tris HCl | pH 7,0 | |
| pH 7,5 | 80 % (v/v) Ethanol | |
| 1 % Ethandiyl-1,2- | 5,5 M Guanidiniumthiocyanat, 40 mM | 34 % |
| bis(dimethyldecylammoniumbromid), 50 mM Tris HCl | Natriumcitrat pH 7,5, 1 % (v/v) Triton® X- | |
| pH 7,5 | 100 98 % (v/v) Ethanol | |
| 1 % Ethandiyl-1,2- | 5,5 M Guanidiniumthiocyanat, 40 mM | 43 % |
| bis(dimethyldecylammoniumbromid), 50 mM Tris HCl | Natriumcitrat pH 7,5, 5 % (v/v) Triton® X- | |
| pH 7,5 | 100 80 % (v/v) Ethanol | |
| 1 % Ethandiyl-1,2- | 5,5 M Guanidiniumthiocyanat, 40 mM | 20 % |
| bis(dimethyldecylammoniumbromid), 50 mM Tris HCl | Natriumcitrat pH 7,5, 1 % (v/v) TWEEN® 20 | |
| pH 7,5 | 80 % (v/v) Ethanol | |
| 1 % Ethandiyl-1,2- | 5,5 M Guanidiniumthiocyanat, 40 mM | 20 % |
| bis(dimethyldecylammoniumbromid), 50 mM Tris HCl | Natriumcitrat pH 7,5, 3 % (v/v) TWEEN® 20 | |
| pH 7,5 | 98 % (v/v) Ethanol | |
| 1 % Ethandiyl-1,2- | 5,5 M Guanidiniumthiocyanat, 40 mM | 56 % |
| bis(dimethyldecylammoniumbromid), 50 mM Tris HCl | Natriumcitrat pH 7,5, 3 % (w/v) | |
| pH 7,5 | ZWITTERGENT® 3-12 98 % (v/v) Ethanol | |
| 1 % Ethandiyl-1,2- | 5,5 M Guanidiniumthiocyanat, 40 mM | 37 % |
| bis(dimethyldecylammoniumbromid), 50 mM Tris HCl | Natriumcitrat pH 7,5, 5 % (w/v) | |
| pH 7,5 | ZWNTERGENT® 3-12 98 % (v/v) Ethanol | |
| 1 % Ethandiyl-1,2- | 5,5 M Guanidiniumthiocyanat, 40 mM | 22 % |
| bis(dimethyldecylammoniumbromid), 50 mM Tris HCl | Natriumcitrat pH 7,5, 1 % (w/v) CHAPSO | |
| pH 7,5 | 98 % (v/v) Ethanol | |
| | | |
| 1 % Ethandiyl-1,2- | 5,5 M Guanidiniumthiocyanat, 40 mM | 19 % |
| bis(dimethyldecylammoniumbromid), 50 mM Tris HCl | Natriumcitrat pH 7,5, 3% (w/v) CHAPSO | |
| pH 7,5 | 98 % (v/v) Ethanol | |

### Beispiel 8: Isolierung von Gesamt-RNA aus HeLa-Zellen

Ein Zellpellet bestehend aus 1 x 10 exp.7 HeLa-Zellen aus einer Suspensionskultur wird in 1 ml einer 2%igen Lösung (w/v) von Ethandiyl-1,2-bis(dimetyldecylammoniumbromid) gepuffert mit einem Tris-HCl Puffer pH 7,0 und versetzt mit 10 µl β-Mercaptoethanol pro ml Lösung aufgenommen, mittels eines Polytron-Homogenisators in einem Eppendorf-Reaktionsgefäß zerkleinert und für 10 min bei Raumtemperatur inkubiert.

Dann wird die Lösung für 3 min bei ca. 1000 x g zentrifugiert. Der Überstand wird abgenommen und das Sediment in 200 µl einer Lösung bestehend aus 4 M Guanidin-thiocyanat, 0,2 M Natriumacetat und 10 % (v/v) Nonidet P40 gelöst. Anschließend werden 100 µl saures Phenol zugegeben und die Lösung wird durch kräftiges Schütteln extrahiert. Nach Zugabe von 100 µl Chloroform wird die Lösung abermals durch kräftiges Schütteln extrahiert und zur Phasentrennung für 1 min. mit 20000 x g zentrifugiert. Die wässerige Phase wird abgenommen und mit 100 µl Chloroform reextrahiert wie oben beschrieben. Die wässerige Phase wird abgenommen und die Nukleinsäuren werden durch Zugabe von 200 µl Isopropanol für 30 min bei -20°C ausgefällt. Die ausgefällten Nukleinsäuren werden durch Zentrifugation für 5 min mit 20000 x g sedimentiert, der Überstand wird abgenommen, das Nukleinsäure-Sediment wird einmal mit einer 80 %igen Ethanol-Lösung gewaschen, getrocknet und in RNase-freiem Aqua dest. gelöst.

Die Menge an isolierter Nukleinsäure wird durch Messung der Lichtabsorption bei einer Wellenlänge von 260 nm ermittelt, und die Reinheit der Nukleinsäure wird durch die Bestimmung des Verhältnisses der Lichtabsorption bei 260 nm und 280 nm ermittelt (s. Tabelle 6).

**Tabelle 6: RNA-Ausbeute und Reinheit bei Einsatz von 1 x 10 exp.7 HeLa-Zellen.**

| | | |
|---|---|---|
| Zur Bestimmung der Ausbeute wird der Berechnungsfaktor für die RNA herangezogen (1 OD₂₆₀nm =40 µg/ml), die OD-Messung erfolgt in Wasser. Es wird eine Dreifachbestimmung durchgeführt. | | |

| Proben Nr. | Ausbeute /µg | OD 260nm/280nm |
|---|---|---|
| 1 | 51,2 | 1,85 |
| 2 | 135 | 1,66 |
| 3 | 77,9 | 1,69 |

Das Ergebnis liegt im Rahmen der Erwartungen an Gesamt-RNA, die man aus 10 exp. 7 HeLa-Zellen isolieren kann.

### Beispiel 9: Isolierung von Gesamt-RNA aus Mausniere

Jeweils 20 mg Nierengewebe wird in 1 ml einer Lösung, die 2 % (w/v) Ethandiyl-1,2-bis(dimetyldecylammoniumbromid) sowie 3 M Urea und 10 µl β-Mercaptoethanol pro ml Lösung enthält und gepuffert wird durch einen 50 mM Tris-HCl Puffer pH 7,0, mittels eines Polytron-Homogenisators in einem Eppendorf-Reaktionsgefäß zerkleinert und anschließend für 10 min bei Raumtemperatur inkubiert. Die Lösung wird dann für 3 min bei ca. 1000 x g zentrifugiert.

Der Überstand wird abgenommen und das Sediment in 200 µl einer Lösung bestehend aus 4 M Guanidin-thiocyanat, 0,2 M Natriumacetat und 10 % (v/v) Nonidet P40 gelöst. Anschließend werden 100 µl saures Phenol zugegeben und die Lösung wird durch kräftiges Schütteln extrahiert. Nach Zugabe von 100 µl Chloroform wird die Lösung abermals durch kräftiges Schütteln extrahiert und zur Phasentrennung für 1 min. mit 20000 x g zentrifugiert. Die wässerige Phase wird abgenommen und mit 100 µl Chloroform reextrahiert wie oben beschrieben. Die wässerige Phase wird abgenommen und die Nukleinsäuren werden durch Zugabe von 200 µl Isopropanol für 30 min bei -20°C ausgefällt. Die ausgefällten Nukleinsäuren werden durch Zentrifugation für 5 min mit 20000 x g sedimentiert, der Überstand wird abgenommen, das Nukleinsäure-Sediment wird einmal mit einer 80 %igen Ethanol-Lösung gewaschen, getrocknet und in RNase-freiem Aqua dest. gelöst.

Die Menge an isolierter Nukleinsäure wird durch Messung der Lichtabsorption bei einer Wellenlänge von 260 nm ermittelt, und die Reinheit der Nukleinsäure wird durch die Bestimmung des Verhältnisses der Lichtabsorption bei 260 nm und 280 nm ermittelt (s. Tabelle 7).

**Tabelle 7: RNA-Ausbeute und Reinheit bei Einsatz von 20 mg Nierengewebe. Zur Bestimmung der Ausbeute wird der Berechnungsfaktor für die RNA herangezogen (1 OD₂₆₀nm =40 µg/ml), die Messung erfolgt in Wasser. Es wird eine Dreifachbestimmung durchgeführt.**

| Proben Nr. | Ausbeute /µg | OD 260nm/280nm |
|---|---|---|
| 1 | 220 | 1,31 |
| 2 | 207 | 1,90 |
| 3 | 256 | 2,26 |

### Beispiel 10

### Aufreinigung von RNA aus Plasma durch eine Komplexierung mit kationischen Verbindungen und anschließender Phenol/Chloroform Extraktion

Als Modell für eine virale RNA (z.B. HCV- oder HIV-RNA) wird HeLa-RNA in eine Mischung aus 140 µl Blutplasma und 140 µl einer Lösung aus 2 % (w/v) Ethandiyl-1,2-bis(dimethyldecylammoniumbromid), gepuffert mit 50 mM Tris-HCl, pH 7,0, gegeben und anschließend für 10 min bei Raumtemperatur inkubiert. Die Lösung wird dann für 3 min bei ca. 1000 x g zentrifugiert.

Der Überstand wird abgenommen und das Sediment in 200 µl einer Lösung bestehend aus 4 M Guanidin-thiocyanat, 0,2 M Natriumacetat und 10 % (v/v) Nonidet P40 gelöst. Anschließend werden 100 µl saures Phenol zugegeben und die Lösung wird durch kräftiges Schütteln extrahiert. Nach Zugabe von 100 µl Chloroform wird die Lösung abermals durch kräftiges Schütteln extrahiert und zur Phasentrennung für 1 min. mit 20000 x g zentrifugiert. Die wässerige Phase wird abgenommen und mit 100 µl Chloroform reextrahiert wie oben beschrieben. Die wässerige Phase wird abgenommen und die Nukleinsäuren werden durch Zugabe von 200 µl Isopropanol für 30 min bei -20°C ausgefällt. Die ausgefällten Nukleinsäuren werden durch Zentrifugation für 5 min mit 20000 x g sedimentiert, der Überstand wird abgenommen, das Nukleinsäure-Sediment wird einmal mit einer 80 %igen Ethanol-Lösung gewaschen, getrocknet und in RNase-freiem Aqua dest. gelöst.

Die Menge an isolierter Nukleinsäure wird durch Messung der Lichtabsorption bei einer Wellenlänge von 260 nm ermittelt, und die Reinheit der Nukleinsäure wird durch die Bestimmung des Verhältnisses der Lichtabsorption bei 260 nm und 280 nm ermittelt (s. Tabelle 8).

Tabelle 8: RNA-Ausbeute und Reinheit. Zur Bestimmung der Ausbeute wird der Berechnungsfaktor für RNA herangezogen (1 OD₂₆₀nm =40 µg/ml), die Messung erfolgt in Wasser. Es wird eine Dreifachbestimmung durchgeführt.

| Proben Nr. | Ausbeute /µg | OD 260nm/280nm |
|---|---|---|
| 1 | 13,3 | 1,73 |
| 2 | 18,7 | 1,72 |
| 3 | 16,4 | 1,91 |

### Beispiel 11:

Isolierung von RNA durch eine Komplexierung mit kationischen Verbindungen und nachfolgender Aufreinigung mittels Membrantechnologien, die in der Patentanmeldung Aktenzeichen PCT/EP98/06756 beschrieben werden.

Je 10 µg RNA in 100 µl Wasser werden mit 100 µl einer 2 %igen Ethandiyl-1,2-bis(dimethyldecylammoniumbromid) Lösung in 50 mM Tris-HCl pH 7,0 versetzt und für 10 min in einem Eppendorf-Reaktionsgefäß bei Raumtemperatur inkubiert. Die Lösung wird anschließend für 3 min bei 20000 x g zentrifugiert, der Überstand dekantiert und das Pellet in 300 µl einer Lösung aus 6 M Guanidinhydrochlorid, 50 mM Tris-HCl, pH 7,0, und 1 % (v/v) Nonidet P40 gelöst. Nach Zugabe von 300 µl einer Lösung aus 80 % Ethanol und 10 % Nonidet P40 (v/v) werden die Ansätze in einer Plastiksäule, die eine Polypropylenfritte zur mechanischen Unterstützung enthält, auf der eine Membran zur Bindung der Nukleinsäuren mittels eines Spannringes fixiert ist, mittels Zentrifugation bei 10000 x g für eine Minute durch die Membran hindurchgeführt. Als Membranen werden eingesetzt:
1. Pall Fluoro Trans G, Polyvinylidendifluorid, hydrophob, Porengröße 0,2 µm
2. GORE-TEX Polyester-Vlies 9318, Polytetrafluorethylen, hydrophil, Porengröße 3 µm
3. Millipore Fluoropore PTFE, Polytetrafluorethylen, hydrophob, Porengröße 3 µm

Der Durchfluß wird in einem Sammelröhrchen aufgefangen und verworfen. Die Membranen werden nacheinander mit 600 µl eines Guanidin-thiocyanat haltigen Puffers und danach mit einem Guanidin-thiocyanat freien Puffer gewaschen. Dabei werden die Waschpuffer jeweils durch Zentrifugation bei 10000 x g durch die Membran geführt. Nach der zweiten Waschung werden die Membranen für 2 min bei 20000 x g trockenzentrifugiert. Dann wird die RNA durch Aufpipettieren von 70 µl Wasser auf die Membran und Inkubation für 2 min bei Raumtemperatur von der Membran eluiert. Das Eluat wird mit einer Pipette von oben von der Membran abpipettiert. Die Elution wird nochmals mit 70 µl Wasser wiederholt, die Eluate werden vereinigt.

Die Menge an isolierter RNA wird durch Messung der Lichtabsorption bei einer Wellenlänge von 260 nm ermittelt, und die Reinheit der RNA wird durch die Bestimmung des Verhältnisses der Lichtabsorption bei 260 nm und 280 nm ermittelt (s. Tabelle 9).

Tabelle 9: RNA-Ausbeute und Reinheit. Zur Bestimmung der Ausbeute wird der Berechnungsfaktor für RNA herangezogen (1 OD260nm =40 µg/ml). Die Messung erfolgt in Wasser. Es werden jeweils Vierfachbestimmungen durchgeführt.

| Membran | Ausbeute /µg | OD 260nm/280nm |
|---|---|---|
| Pall Fluoro Trans G | 4,95 | 1,96 |
| | 2,32 | 1,99 |
| | 4,73 | 1,96 |
| | 2,49 | 1,98 |
| GORE-TEX Polyester Vlies 9318 | 3,25 | 1,90 |
| | 3,07 | 1,80 |
| | 1,52 | 1,66 |
| | 2,57 | 1,88 |
| Millipore Fluoropore PTFE | 4,32 | 1,99 |
| | 7,08 | 1,99 |
| | 7,66 | 1,97 |
| | 7,25 | 1,98 |

### Beispiel 12: Stabilisierung von RNA in Blut mittels kationischer Verbindungen mit zwei oder mehr Ammoniumzentren

Jeweils 200 µl frisches Blut werden mit 600 µl einer Lösung aus:
2 % (w/v) Ethandiyl-1,2-bis(dimethyldecylammoniumbromid) in 200 mM Natriumcitrat pH 3,0
2 % (w/v) Propandiyl-1,2-bis(dimethyldecylammoniumbromid) in 200 mM Natriumcitrat pH 3,0
2 % (w/v) Ethandiyl-1,2-bis(dimethyltetradecylammoniumbromid) in 200 mM Natriumcitrat pH 3,0
2 % (w/v) N,N',N''-tridecyl-N,N,N',N'',N''-pentamethyl-bis-(2-ammonioethyl)ammoniumbromid
in 200 mM Natriumcitrat pH 3,0
versetzt und für 48 h bei Raumtemperatur gelagert. Alle Ansätze wurden als Doppelbestimmungen durchgeführt.

Zur Isolierung der RNA werden die Proben für 2 min bei 1000 x g zentrifugiert, der Überstand dekantiert und das Pellet in 700 µl einer Lösung aus 6 M Guanidinhydrochlorid, 200 mM Tris-HCl pH 7,0 und 1 % (v/v) Nonidet P40 gelöst. Dann werden 80 µg Proteinase K zugegeben und die Ansätze werden für 30 min bei 40°C inkubiert. Es werden jeweils 350 µl saures Phenol zugegeben und die Ansätze werden durch kräftiges Schütteln extrahiert. Nach Zugabe von 350 µl Chloroform und erneuter Extraktion werden die Ansätze zur Trennung der Phasen für 3 min bei 14000 x g zentrifugiert. Die wässerige Phase wird abgenommen und nochmals mit 700 µl Chloroform extrahiert. Nach erneuter Zentrifugation wird wieder die wässerige Phase abgenommen und die RNA durch Zugabe von 70 µl 3 M Natriumacetat pH 5,2 und 700 µl Isopropanol für 30 min bei -20°C ausgefällt. Die RNA wird für 10 min bei 20000 x g abzentrifugiert, der Überstand abgenommen, das Pellet einmal mit 600 µl 80 % (v/v) Ethanol gewaschen, anschließend getrocknet und in 100 µl RNase freiem Wasser zurückgelöst.

Die Menge an isolierter RNA wird durch Messung der Lichtabsorption bei einer Wellenlänge von 260 nm ermittelt, und die Reinheit der RNA wird durch die Bestimmung des Verhältnisses der Lichtabsorption bei 260 nm und 280 nm ermittelt (s. Tabelle 10).

**Tabelle 10: RNA-Ausbeute und Reinheit. Zur Bestimmung der Ausbeute wird der Berechnungsfaktor für RNA herangezogen (1 OD₂₆₀nm =40 µg/ml). Es werden jeweils. Doppelbestimmungen durchgeführt.**

| Kationische Verbindung | Ausbeute /µg | OD 260nm/280nm |
|---|---|---|
| Ethandiyl-1,2-bis(dimethyldecytammoniumbromid) | 0,36 | 1,24 |
| | 0,60 | 1,14 |
| Propandiyl-1,2-bis(dimethyldecylammoniumbromid) | 3,2 | 1,01 |
| | 0,72 | 1,1 |
| Ethandiyl-1,2-bis(dimethyltetradecylammoniumbromid) | 0,96 | 1,15 |
| | 1,2 | 1,05 |
| N,N',N"-tridecyl-N,N,N',N",N"-pentamethyl-bis-(2-ammonioethyl)ammoniumbromid | 0,72 | 1,17 |
| | 1,8 | 0,72 |

### Beispiel 13: Isolierung von RNA mittels kationischer Substanzen mit zwei oder mehr Ammoniumzentren

Je 25 µg reine HeLa-RNA, gelöst in 140 µl Wasser, wird mit 140 µl der Substanzen in unterschiedlicher Konzentration von 1 - 15 % (w/v), gelöst in Wasser, versetzt und bei RT für 10 min inkubiert, die Substanz-RNA-Komplexe werden für 10 min bei 5000 x g abzentrifugiert, in 150 µl eines Puffers bestehend aus 3,5 M Guanidiniumthiocyanat, 25 mM Natriumcitrat pH 7,5 aufgenommen und nach dem folgenden Protokoll aufgereinigt. Zur Probe werden 150 µl 70% Ethanol zugegeben. Dann wird die Probe mittels Vakuum auf eine Silica-Membran enthaltende spin-Säule aufgetragen. Die spin-Säule wird zweimal mit einem Ethanol und NaCl haltigen Waschpuffer gewaschen, wobei der Waschpuffer ebenfalls mittels Vakuum durch die Membran geführt wird. Die spin-Säule wird mittels Vakuum für 10 min getrocknet. Dann wird die RNA zweimal mit je 60 µl Wasser eluiert, wobei die spin-Säule für je 1 min bei 10 000 x g zentrifugiert wird. Die Ergebnisse werden in Tabelle 11 zusammengefaßt.

**Tabelle 11: Ausbeute an HeLa-RNA im Eluat in Abhängigkeit von der Konzentration der eingesetzten Substanz. Zur Bestimmung der Ausbeute wird der Berechnungsfaktor für RNA herangezogen (1 OD260nm =40 µg/ml).**

| **Substanz** | **Konzentration in % (w/v)** | **Ausbeute/** µ**g** |
|---|---|---|
| Ethandiyl-1,2-bis(dimethyldecylammoniumbromid) | 1 | 22,2 |
| | 9 | 25,0 |
| | 15 | 19,5 |
| Ethandiyl-1,2-bis(dimethyldecylammoniumthiosulfat) | 1 | 24,5 |
| | 9 | 25 |
| | 15 | 24 |
| Ethandiyl-1,2-bis(dimethyldecylammoniumsulfat) | 1 | 22,2 |
| | 9 | 25 |
| | 15 | 22,4 |
| Ethandiyl-1,2-bis(dimethyldecylammoniumjodid) | 1 | 18,9 |
| | 9 | 23 |
| | 15 | 19,2 |
| N,N',N"-tridecyl-N,N,N',N",N"-pentamethyl-bis-(2-ammonioethyl)ammoniumbromid | 1 | 15,3 |
| | 9 | 12,7 |
| | 13 | 23,2 |
| N,N',N"-tritetradecyl-N,N,N',N",N"-pentamethyl-bis-(2-ammonioethyl)ammoniumbromid | 1 | 11,3 |
| | 9 | 9,9 |
| | 15 | 6,3 |
| Ethandiyl-1,2-bis(dimethyloctylammoniumbromid) | 3 | 7,6 |
| | 15 | 6,5 |
| Propandiyl-1,2-bis(dimethyldecylammoniumbromid) | 3 | 21,2 |
| | 8 | 24,6 |
| | 13 | 24,7 |
| Butandiyl-1,2-bis(dimethyldecylammoniumbromid) | 1 | 24,6 |
| | 9 | 25 |
| | 13 | 11,3 |
| Ethandiyl-1,2-bis(dimethyldodecylammoniumbromid) | 1 | 14,4 |
| | 8 | 14,5 |
| | 15 | 7,3 |
| Propandiyt-1,2-bis(dimethyitetradecylammoniumbromid) | 1 | 13,8 |
| | 9 | 18 |
| | 15 | 14,6 |
| Hexadimethrin Bromid | 1 | 9,6 |
| | 5 | 3 |

Das Ergebnis zeigt, daß alle Substanzen zur Komplexierung von RNA verwendet werden können. Einige Substanzen wirken aber unter den gewählten Bedingungen deutlich effektiver als andere.

### Beispiel 14: Stabilisierung von RNA mittels kationischer Verbindungen in Blut

Zu 1 ml Blut wird 1 ml Stabilisierungspuffer, bestehend aus 2 % (w/v) Ethandiyl-1,2-bis(dimethyldecylammoniumbromid), 50 mM Kaliumacetat pH 5,5 und 50 mM Tris HCl pH 7,0 gegeben. Der Ansatz wird gut gemischt und für 24 Stunden bzw. 96 Stunden bei Raumtemperatur oder bei 4°C gelagert. Die Komplexe bestehend aus Nukleinsäure und kationischer Verbindung werden für 3 min bei 4000 x g zentrifugiert, der Überstand entfernt und das Pellet in 1 ml eines Puffers bestehend aus 6 M Guanidinhydrochlorid, 50 mM Tris HCl pH 7,0, 1 % (v/v) Nonidet P40 rückgelöst. Dann werden 800 µg Proteinase K zugegeben und der Ansatz für 1 Stunde bei 60°C inkubiert. Dann wird 1 ml 80 % (v/v) Ethanol, 10 % (v/v) Nonidet P40 zugegeben und die Probe mittels Vakuum auf eine Silica-Membran enthaltende spin-Säule aufgetragen. Die spin-Säule wird mit 350 µl eines Guanidiniumthiocyanat und Ethanol-haltigen Puffers gewaschen. Dann werden 80 µl eines Tris HCl-Puffers mit MgCl_{2,} der 75 U DNase I (Pharmacia) enthält, auf die Silica-Membran pipettiert und zum Abbau der genomischen DNA für 15 min bei Raumtemperatur inkubiert. Die spin-Säule wird nochmals mit 350 µl des Guanidiniumthiocyanat und Ethanol-haltigen Puffers gewaschen und im Anschluß mit 700 µl eines Ethanol-haltigen Waschpuffers. Die spin-Säule wird für 3 min bei 20 000 x g trockenzentrifugiert und die RNA in zwei Schritten mit je 30 µl Wasser eluiert.

Jeweils 3 µl des Eluates werden für einen RT-PCR Nachweis der β-Actin mRNA in einem ABI PRISM 7700 Sequence Detector (Applied Biosystems) (sog. TaqMan Technologie) eingesetzt.

Die TaqMan Technologie nutzt Oligonukleotid-Sonden, die einen Reporter- und einen Quencher-Farbstoff enthalten. Während der PCR-Amplifikation wird die 5'-3' Exonuklease Aktivität der Taq-Polymerase genutzt um den Reporter-Farbstoff vom Quencher-Farbstoff zu trennen, wodurch ein Sequenzspezifisches Fluoreszenz-Signal generiert wird, das mit jedem Amplifikationszyklus ansteigt. Die Quantifizierung basiert auf dem threshold cycle, bei dem ein vorher definierter Grenzwert der Fluoreszenz erreicht wird. Der Vergleich der threshold cycles liefert ein Maß für die relative Konzentration an template in verschiedenen Proben. Die Messung während der logarithmischen Phase, wenn die PCR Genauigkeit am höchsten ist, liefert präzise Daten für eine genaue Bestimmung.

Das Ergebnis ist in Tabelle 12 dargestellt.

**Tabelle 12: Analyse der β-Actin mRNA mittels TaqMan^{™} RT-PCR. Dargestellt sind die threshold cycles (C_{T}) der TaqMan^{™} Auswertung in Abhängigkeit von der Lagerung der stabilisierten Probe. Es wurden jeweils Doppelbestimmungen von jeder Probe im ABI PRISM 7700 Sequence Detector durchgeführt.**

| **Lagerung** | **C_{T}** |
|---|---|
| 24 h 4°C | 17,23 |
| 96 h 4°C | 18,51 |
| | 18,30 |
| | 18,29 |
| 24 h Raumtemperatur | 17,93 |
| | 17,89 |
| 96 h Raumtemperatur | 19,34 |
| | 19,35 |

### Beispiel 15: Stabilisierung von RNA mittels kationischer Verbindungen in Plasma

In einem 2 ml Reaktionsgefäß werden zu 500 µl Plasma 500 µl enthaltend 2 % (w/v) Ethandiyl-1,2-bis(dimethyldecylammonium-bromid), 200 mM Natriumcitrat pH 3,0 zugegeben. In den Deckel des Reaktionsgefäßes werden 15 µg HeLa-RNA pipettiert, der Deckel geschlossen und der Ansatz gemischt. Jeweils eine Probe wird für 10 min bei Raumtemperatur inkubiert und danach sofort weiterverarbeitet. Die anderen Proben werden für 24 und 48 Stunden bei 4°C gelagert und dann die RNA isoliert. Zur Kontrolle wird die HeLa-RNA direkt ins Plasma pipettiert und nach 10 Sekunden werden 500 µl 2 % (w/v) Ethandiyl-1,2-bis(dimethyldecylammoniumbromid), 200 mM Natriumcitrat pH 3,0 zugegeben und weitere 10 min bei Raumtemperatur inkubiert, bevor die Probenvorbereitung durchgeführt wird, bzw. die Kontrollen werden zusammen mit den stabilisierten Proben für 24 und 48 Stunden bei 4°C gelagert. Zur Probenvorbereitung werden dann 500 µl 2 % (w/v) Ethandiyl-1,2-bis(dimethyldecylammoniumbromid), 200 mM Natriumcitrat pH 3,0 zugegeben, der Ansatz für 10 min bei Raumtemperatur inkubiert und dann weiterverarbeitet.

Die Komplexe bestehend aus RNA und kationischer Verbindung werden für 3 min bei ca. 1100 x g abzentrifugiert, der Überstand abgenommen und das Pellet in 600 µl eines Puffers bestehend aus 6 M Guanidinhydrochlorid, 50 mM Tris HCl pH 7,0, 1 % (v/v) Nonidet P40 rückgelöst. Dann werden 800 µg Proteinase K zugegeben und der Ansatz für 30 min bei 40°C inkubiert. Dann wird 600 µl 80 % (v/v) Ethanol, 10 % (v/v) Nonidet P40 zugegeben und die Probe auf eine Silica-Membran enthaltende spin-Säule aufgetragen, wobei sie mittels Zentrifugation bei 3700 x g für 1 min durch die Membran geführt wird.

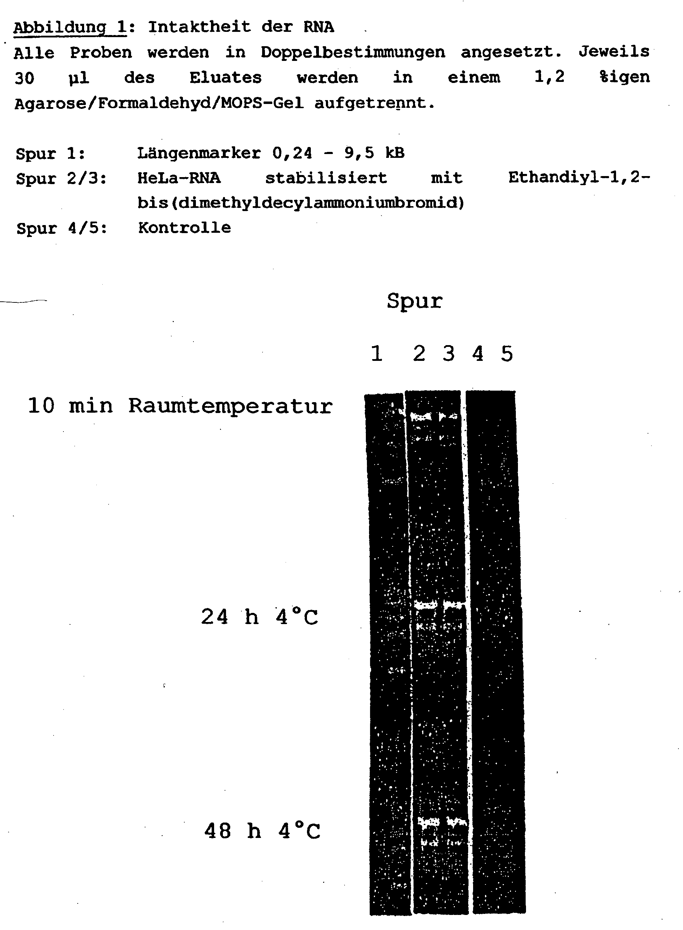

Die spin-Säule wird mit 350 µl eines Guanidiniumthiocyanat und Ethanol haltigen Puffers gewaschen. Dann werden 80 µl eines Tris HCl-Puffers mit MgCl2, der 75 U DNase I (Pharmacia) enthält auf die Silica-Membran pipettiert und zum Abbau der genomischen DNA für 15 min bei Raumtemperatur inkubiert. Die spin-Säule wird nochmals mit 350 µl des Guanidiniumthiocyanat und Ethanol haltigen Puffers gewaschen und im Anschluß zweimal mit je 500 µl eines Ethanol haltigen Waschpuffers. Die spin-Säule wird für 3 min bei 20 000 x g trockenzentrifugiert und die RNA in zwei Schritten mit je 50 µl Wasser eluiert. Jeweils 4 µl des Eluates werden für einen RT-PCR Nachweis der β-Actin mRNA in einem ABI PRISM 7700 Sequence Detector (Applied Biosystems) eingesetzt. Die Reaktionsbedingungen für den RT-PCR Nachweis sind identisch mit den in Beispiel 12 beschriebenen. Jeweils 30 µl des Eluates werden in einem 1,2 %igen Agarose/Formaldehyd/MOPS-Gel aufgetrennt. Die Ergebnisse sind in Tabelle 13 und Abbildung 1 dargestellt.

**Tabelle 13: Analyse der β-Actin mRNA mittels TaqMan^{™} RT-PCR. Dargestellt sind die threshold cycles (Ct) der TaqMan^{™} Auswertung in Abhängigkeit von der Lagerungsdauer der stabilisierten Proben und der Kontrollen. Es werden jeweils Doppelbestimmungen von jeder Probe im ABI PRISM 7700 Sequence Detector durchgeführt.**

| **Lagerung** | **stab. Probe** | **Kontrolle** |
|---|---|---|
| 10 min RT* | 15,90 | 21,49 |
| | 16,17 | 22,16 |
| | | |
| 24 h 4°C | 16,25 | 40** |
| | 15,82 | 40** |
| | | |
| 48 h 4°C | 16,43 | 40** |
| | 16,49 | 40** |

| | | |
|---|---|---|
| * Bei der Kontrolle wird die RNA für ca. 10 Sekunden ungeschützt im Plasma inkubiert, bevor der Stabilisierungspuffer zugegeben wird und für weitere 10 min bei Raumtemperatur inkubiert wird. ** Es erfolgt innerhalb der 40 Zyklen keine Amplifikation von β-Aktin mRNA. | | |

In den Beispielen 14 und 15 wurde die mRNA des β-Aktin Gens mittels Amplifikation in einem ABI PRISM 7700 Sequence Detector nachgewiesen.

Die β-Aktin mRNA wurde in einer Ein-Gefäß TaqMan RT-PCR amplifiziert. Für den 25 µl Reaktionsansatz wurden Standardreagenzien in Kitformat der Firma Perkin Elmer Applied Biosystems, (TaqMan PCR Reagent Kit, β-Aktin Detection Kit, AmpliTaq Gold DNA Polymerase, MuLV Reverse Transkriptase) sowie der Firma Promega (RNasin) eingesetzt. Die cDNA wurde für 60 min. bei 37°C synthetisiert, die AmpliTaq Gold DNA Polymerase anschließend für 12 min. bei 95°C aktiviert. Das spezifische β-Actin Fragment wurde mittels sich direkt anschließender PCR amplifiziert. Dafür wurden 40 PCR-Zyklen durchgeführt mit jeweils 15 sec 95°C und 1 min 60°C.

Im Beispiel 15 bedeutet der Anstieg des C_{T}-Wertes (Threshold Cycle Wert) der Kontrollprobe "10 min. RT", zu der erst nach 10 sec der Stabilisierungspuffer gegeben wurde, von ca. 16 auf ca. 22, daß während der 10 Sekunden, in denen die RNA ungeschützt im Plasma vorlag, mehr als 99% der RNA abgebaut wurde (Kontrolle 10 min. RT). Dabei wurde davon ausgegangen, daß eine Differenz von 1 Threshold Cycle (1 C_{T}) einen ca. 2-fachen Unterschied der β-Aktin-mRNA Mengen in den zu analysierenden Proben bedeutet. Dieses Ergebnis wird durch die Gelanalyse bestätigt, die bei der Kontrolle nur noch einen sehr schwachen Schmier sehr stark abgebauter RNA zeigt (Abbildung 1, Spuren 4 und 5, 10 min. Raumtemperatur). Nach einer längeren Lagerung von 24 h bzw. 48 h ohne Zugabe von Stabilisierungspuffer ist die RNA in den Kontrollen komplett abgebaut (Kontrolle 24 h, 4°C und Kontrolle 48 h, 4°C). Es ist keine RNA mehr nachweisbar, weder in einer Agarose/Formaldehyd-Gelelektrophorese (Abb. 1, Spuren 4 und 5) noch in einer β-Aktin TaqMan RT-PCR (Kontrollen). Dabei bedeutet ein Threshold von 40, daß während der 40 PCR-Zyklen kein Amplifikationssignal erzeugt wurde und somit keine β-Aktin-mRNA mehr nachgewiesen werden konnte.

Demgegenüber zeigen sowohl die Ergebnisse der β-Aktin-mRNA Amplifikation, als auch die Ergebnisse der Gelanalyse (Abb. 1), daß bei den gelagerten Proben, zu denen Stabilisierungspuffer gegeben wurde, kein Abbau der RNA stattgefunden hat (stab. Proben 10 min. RT, 24 h 4°C, 48 h 4°C). Dies ist an den deutlich erkennbaren Banden der ribosomalen RNA im Gel als auch an den im Rahmen der Genauigkeit der Methode als unverändert zu bezeichnenden C_{T}-Werten der TaqMan RT-PCR zu erkennen.

Dieses Ergebnis wird auch im Beispiel 14 bestätigt, wo in den Blutproben selbst nach Lagerung für 96 h bei Raumtemperatur noch ein sehr sensitiver Nachweis der β-Aktin mRNA mittels der TaqMan^{™} RT-PCR möglich ist.

Es wurde sowohl für Plasma als auch für Blut gezeigt, daß RNA in diesen biologischen Proben durch den Einsatz kationischer Verbindungen vor einem Abbau geschützt werden kann. Ungeschützte RNA wird hingegen in beiden Probenmaterialien innerhalb von wenigen Sekunden vollständig abgebaut.

### Beispiel 16: Isolierung von HeLa-RNA aus Plasma mittels Ethandiyl-1,2-bis(dimethyldecylammoniumbromid), gepuffert mit Zitronensäure im pH Bereich von pH 3-7

15 µg HeLa-RNA werden in 500 µl Plasma, gemischt mit 500 µl eines Puffers, enthaltend 2 % (w/v) Ethandiyl-1,2-bis(dimethyldecylammoniumbromid) und 0,5 M Zitronensäure, unterschiedlicher pH-Werte (pH 3-7) pipettiert und für 10 Minuten bei Raumtemperatur inkubiert. Zur Isolierung der RNA werden die Komplexe bestehend aus kationischer Verbindung und Nukleinsäure für 3 min bei 1100 x g abzentrifugiert und in 600 µl eines Puffers enthaltend 6 M Guanidinhydrochlorid, 1 % (v/v) Nonidet-P40 und 50 mM Tris HCL pH 7,0 aufgenommen. Dann werden 800 µg Proteinase K zugegeben und der Ansatz für 30 min bei 40°C inkubiert, mit 600 µl einer Lösung enthaltend 80 % (v/v) Ethanol und 10 % (v/v) Nonidet-P40 versetzt und anschließend auf eine Silica-Membran enthaltende spin-Säule aufgetragen. Die Probe wird mittels Zentrifugation durch die Membran geführt. Die spin-Säule wird einmal mit einem Guanidinthiocyanat- und Ethanol-haltigen Waschpuffer gewaschen und zweimal mit einem NaCl- und Ethanol-haltigen Waschpuffer. Die spin-Säule wird für 3 min bei 20000 x g trockenzentrifugiert. Die RNA wird anschließend in 100 µl RNase-freiem Wasser, das ebenfalls mittels Zentrifugation durch die Membran geführt wird, eluiert. Jeweils 30 µl des Eluates werden auf einem 1,2 %igen Agarose/Formaldehyd Gel aufgetrennt.

Zur Kontrolle (K) wird die HeLa RNA direkt in das Plasma pipettiert und nach 10 Sekunden werden 500 µl eines Puffers, der neben 2 % (w/v) Ethandiyl-1,2-bis(dimethyldecylammonium-bromid) noch 0,5 M Zitronensäure pH 3,0 enthält, zugegeben und für weitere 10 min inkubiert. Danach wird die RNA isoliert wie oben beschrieben.

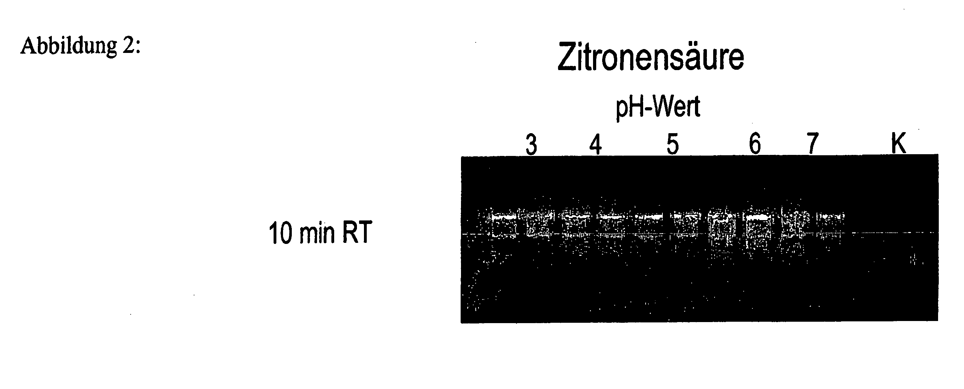

Abbildung 2 zeigt ein Agarosegel der oben beschriebenen Reaktionsansätze. Die Zahlen über den Spuren geben die jeweiligen pH-Werte an und K steht für Kontrollversuch.

Der Versuch zeigt klar, daß über den gesamten pH-Bereich intakte RNA mit gleicher Effizienz isoliert werden kann. Demgegenüber zeigt die Kontrolle, daß ungeschützte RNA im Plasma binnen Sekunden abgebaut wird.

### Beispiel 17: Stabilisierung von HeLa-RNA in Plasma mittels Ethandiyl-1,2-bis(difmethyldecylammoniumbrmid), gepuffert mit Zitronensäure im pH-Bereich von 3-5

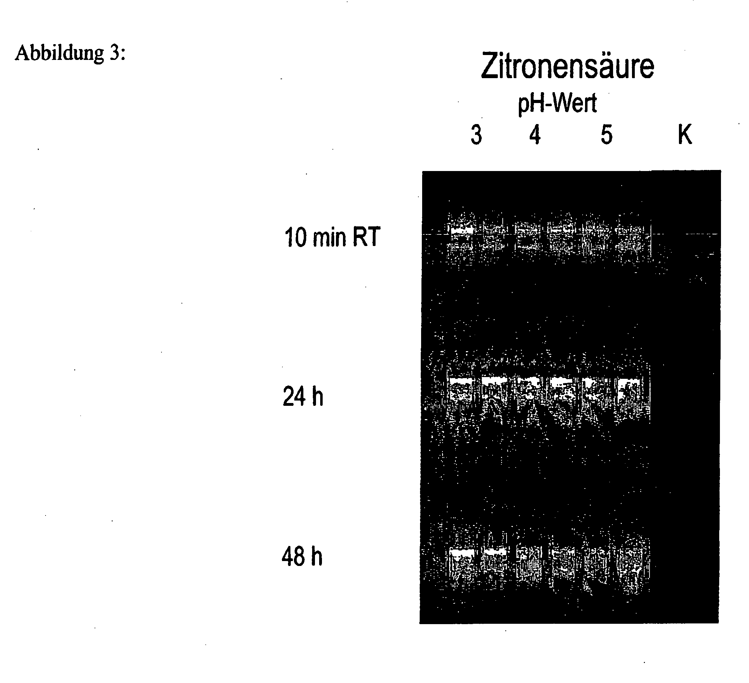

15 µg HeLa-RNA werden in 500 µl Plasma, stabilisiert mit 500 µl eines Puffers, enthaltend 2 % (w/v) Ethandiyl-1,2-bis(dimethyldecylammoniumbromid) und 0,5 M Zitronensäure, unterschiedlicher pH-Werte (pH 3-5) für 10 Minuten bei Raumtemperatur, bzw. für 24 und 48 Stunden bei 4°C gelagert. Zur Isolierung der RNA werden die Komplexe bestehend aus kationischer Verbindung und Nukleinsäure für 3 min bei 1100 x g abzentrifugiert und in 600 µl eines Puffers enthaltend 6 M Guanidinhydrochlorid, 1 % (v/v) Nonidet-P40 und 50 mM Tris HCL pH 7,0 aufgenommen. Dann werden 800 µg Proteinase K zugegeben und der Ansatz für 30 min bei 40°C inkubiert, mit 600 µl einer Lösung enthaltend 80 % (v/v) Ethanol und 10 % (v/v) Nonidet-P40 versetzt und anschließend auf eine Silica-Membran enthaltende spin-Säule aufgetragen. Die Probe wird mittels Zentrifugation durch die Membran geführt. Die spin-Säule wird einmal mit einem Guanidinthiocyanat- und Ethanol-haltigen Waschpuffer gewaschen und zweimal mit einem NaCl-und Ethanol-haltigen Waschpuffer. Die spin-Säule wird für 3 min bei 20000 x g trockenzentrifugiert. Die RNA wird anschließend in 100 µl RNase-freiem Wasser, das ebenfalls mittels Zentrifugation durch die Membran geführt wird, eluiert. Jeweils 30 µl des Eluates werden auf einem 1,2 %igen Agarose/Formaldehyd Gel aufgetrennt (Abb.3).

Zur Kontrolle (K) wird die HeLa RNA direkt in das Plasma pipettiert und nach 10 Sekunden werden 500 µl eines Puffers, der neben 2 % (w/v) Ethandiyl-1,2-bis(dimethyldecylammoniumbromid) noch 0,5 M Zitronensäure pH 3,0 enthält, zugegeben und für weitere 10 min inkubiert. Danach wird die RNA isoliert wie oben beschrieben.

Abbildung 3 zeigt ein Agarosegel der Reaktionsansätze bei einer Stabilisierung von 10 Minuten bei Raumtemperatur, bzw. von 24 und 48 Stunden bei 4°C. Die Zahlen über den Spuren geben die jeweiligen pH-Werte an und K steht für Kontrollversuch.

Der Versuch zeigt, daß durch den Puffer, enthaltend Ethandiyl-1,2-bis(dimethyldecylammoniumbromid) und Zitronensäure, RNA in Plasma auch über einen langen Zeitraum stabil bleibt.

### Beispiel 18: Isolierung von HeLa-RNA aus Plasma mittels kationischer Verbindungen mit zwei Stickstoff- oder Phosphor-Zentren, verknüpft über eine Brücke bestehend aus einer aromatischen Verbindung oder Ethan

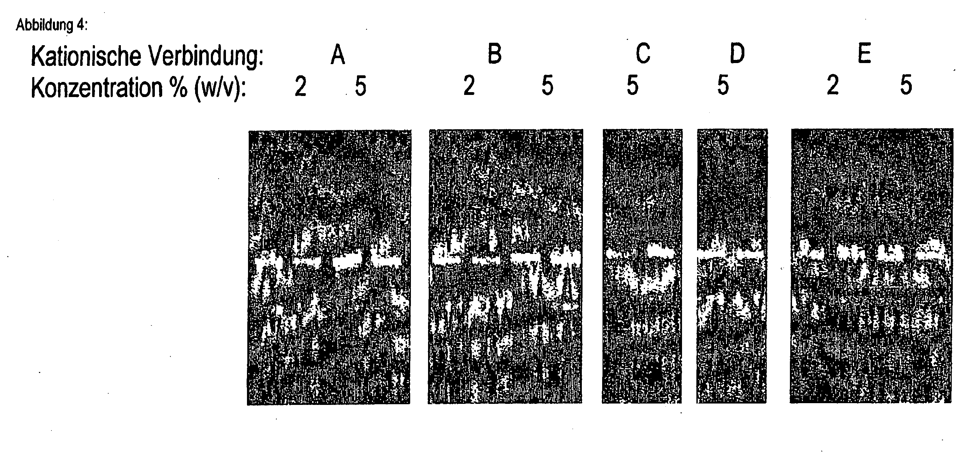

5 µg HeLa-RNA werden in 500 µl Plasma, gemischt mit 500 µl einer Lösung der kationischen Verbindung A, B, C, D oder E (siehe unten), pipettiert und anschließend für 10 min bei Raumtemperatur gelagert. Zur Isolierung der RNA werden die Komplexe bestehend aus kationischer Verbindung und Nukleinsäure für 3 min bei 1530 x g abzentrifugiert und in 300 µl eines Puffers enthaltend 6 M Guanidinhydrochlorid, 1 % (v/v) Nonidet-P40 und 50 mM Tris HCL pH 7,0 aufgenommen. Dann werden 400 µg Proteinase K zugegeben und der Ansatz für 10 min bei 40°C inkubiert, mit 300 µl einer Lösung enthaltend 80 % (v/v) Ethanol und 10 % (v/v) Nonidet-P40 versetzt und anschließend auf eine Silica-Membran enthaltende spin-Säule aufgetragen. Die Probe wird mittels Zentrifugation durch die Membran geführt. Die spin-Säule wird einmal mit einem Guanidinthiocyanat- und Ethanol-haltigen Waschpuffer gewaschen und zweimal mit einem NaCl- und Ethanol-haltigen Waschpuffer. Die spin-Säule wird für 3 min bei 20000 x g trockenzentrifugiert. Die RNA wird anschließend in 80 µl RNase-freiem Wasser, das ebenfalls mittels Zentrifugation durch die Membran geführt wird, eluiert. Jeweils 25 µl des Eluates werden auf einem 1,2 %igen Agarose/Formaldehyd Gel aufgetrennt (Abb. 4).

Abbildung 4 zeigt 5 Agarosegele der o.g. Reaktionsansätze. Die Buchstaben und Zahlen über den Gelen geben die Konzentration der jeweiligen kationischen Verbindungen A bis E an, wobei:
A: o-Xylylen-bis-decyldimethylammonium bromid
B: m-Xylylene-bis-decyldimethylammonium bromid
C: p-Xylylen-bis-decyldimethylammonium bromid
D: [1,8]-dimethylnaphthaleno,alpha,alpha'-bis-dimethyldecylammonium bromid und
E: Ethandiyl-1,2-bis(decyldimethylphosphonium bromid)
ist. Der Versuch zeigt, daß die o.g. kationischen Verbindungen zur Isolierung von RNA aus Plasma geeignet sind. Die Ausbeuten liegen zwischen 63 % (=3,2 µg) und 74 % (3,7 µg) der eingesetzten RNA.

### Beispiel 19: Isolierung von RNA und genomischer DNA aus 1 x 10⁶ HeLa Zellen mittels kationischer Verbindungen mit zwei Stickstoff- oder Phosphor-Zentren, verknüpft über eine Brücke bestehend aus einer aromatischen Verbindung oder Ethan

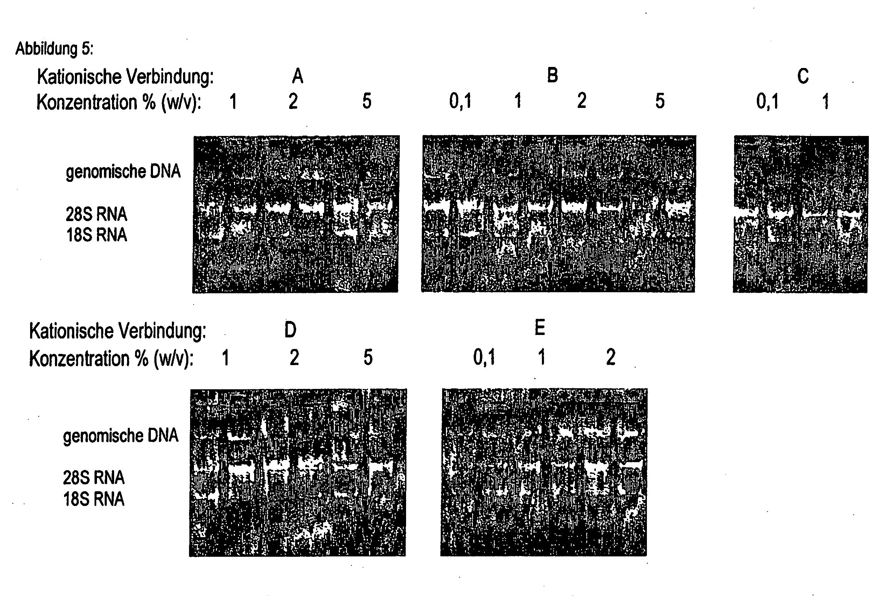

1 x 10⁶ HeLa Zellen, gelöst in 500 µl PBS-Puffer, werden mit 500 µl einer Lösung der kationischen Verbindung A,B,C,D oder E (siehe unten) gemischt und anschließend für 10 min bei Raumtemperatur gelagert. Zur Isolierung der RNA werden die Komplexe bestehend aus kationischer Verbindung und Nukleinsäure für 3 min bei 1530 x g abzentrifugiert und in 300 µl eines Puffers enthaltend 6 M Guanidinhydrochlorid, 1 % (v/v) Nonidet-P40 und 50 mM Tris HCL pH 7,0 aufgenommen. Dann werden 400 µg Proteinase K zugegeben und der Ansatz für 10 min bei 40°C inkubiert, mit 300 µl einer Lösung enthaltend 80 % (v/v) Ethanol und 10 % (v/v) Nonidet-P40 versetzt und anschließend auf eine Silica-Membran enthaltende spin-Säule aufgetragen. Die Probe wird mittels Zentrifugation durch die Membran geführt. Die spin-Säule wird einmal mit einem Guanidinthiocyanat- und Ethanol-haltigen Waschpuffer gewaschen und zweimal mit einem NaCl- und Ethanol-haltigen Waschpuffer. Die spin-Säule wird für 3 min bei 20000 x g trockenzentrifugiert. Die RNA wird anschließend in 80 µl RNase-freiem Wasser, das ebenfalls mittels Zentrifugation durch die Membran geführt wird, eluiert. Jeweils 25 µl des Eluates werden auf einem 1,2 %igen Agarose/Formaldehyd Gel aufgetrennt (Abb. 5).

Abbildung 5 zeigt 5 Agarosegele der o.g. Reaktionsansätze. Die Buchstaben und Zahlen über den Gelen geben die Konzentration der jeweiligen Kationischen Verbindungen A bis E an, wobei:
A: o-Xylylen-bis-decyldimethylammonium bromid
B: m-Xylylen-bis-decyldimethylammonium bromid
C: p-Xylylen-bis-decyldimethylammonium bromid
D: [1,8]-dimethylnaphthaleno,alpha,alpha'-bis-dimethyldecylammonium bromid und
E: Ethandiyl-1,2-bis(decyldimethylphosphonium bromid)
ist.

### Beispiel 20: Isolierung genomischer DNA aus Blut mittels kationischer Verbindungen mit zwei Stickstoff- oder Phosphor-Zentren, verknüpft über eine Brücke bestehend aus einer aromatischen Verbindung oder Ethan

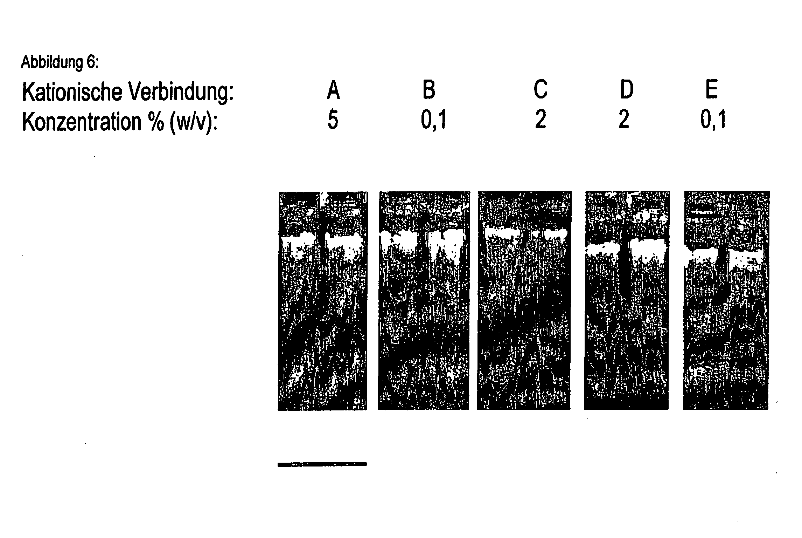

0,5 ml Blut werden mit 0,5 ml der kationischen Verbindung A,B,C,D oder E (siehe unten) gemischt und anschließend für 10 min bei Raumtemperatur gelagert. Zur Isolierung der DNA werden die Komplexe aus kationischer Verbindung und DNA für 3 min bei 1530 x g abzentrifugiert. Das Pellet wird in 360 µl eines NaCl- und EDTA-haltigen Puffers aufgenommen, dann werden 400 µl Puffer AL (QIAGEN GmbH; Cat. No.: 19075) sowie 20 µl Proteinase K (18 mg/ml) zugegeben. Die Proben werden für 10 min bei 65°C inkubiert, dann werden 420 µl Ethanol zugegeben und die Probe auf eine Silica-Membran enthaltende spin-Säule aufgetragen. Die Probe wird mittels Zentrifugation durch die Membran geführt. Die Silica-Membran wird je einmal mit Puffer AW 1 (QIAGEN GmbH, Cat. No.: 19081) und einmal mit Puffer AW 2 (QIAGEN GmbH, Cat. No.: 19072) gewaschen. Die DNA wird mit 100 µl Wasser eluiert. Je 25 µl des Eluates werden auf einem 0,8 %igen Agarose/TBE Gel aufgetrennt (Abb. 6).

Abbildung 6 zeigt 5 Agarosegele der o.g. Reaktionsansätze. Die Buchstaben und Zahlen über den Gelen geben die Konzentration der jeweiligen Kationischen Verbindungen A bis E an, wobei:
A: o-Xylylen-bis-decyldimethylammonium bromid
B: m-Xylylen-bis-decyldimethylammonium bromid
C: p-Xylylen-bis-decyldimethylammonium bromid
D: [1,8]-dimethylnaphthaleno,alpha,alpha'-bis-dimethyldecylammonium bromid und
E: Ethandiyl-1,2-bis(decyldimethylphosphonium bromid)
ist. Die Ausbeuten an genomischer DNA aus 0,5 ml Blut liegen zwischen 6 µg und 11 µg.

### Beispiel 21: Stabilisierung von RNA in Plasma mittels kationischer Verbindungen mit zwei Stickstoff- oder Phosphor-Zentren, verknüpft über eine Brücke bestehend aus einer aromatischen Verbindung oder Ethan, gepuffert mit einem Weinsäurepuffer im pH Bereich 4-5

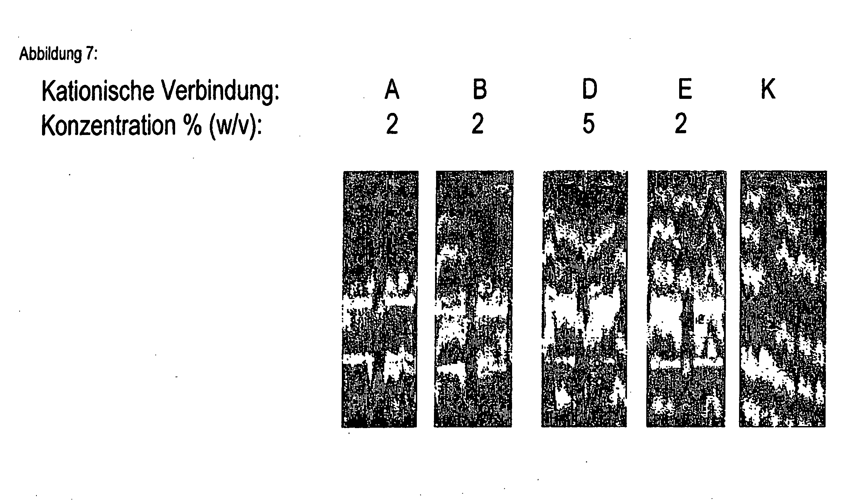

6 µg HeLa-RNA werden in 500 µl Plasma, stabilisiert mit 500 µl eines Puffers, enthaltend die kationische Verbindung A, B, D oder E in einer Konzentration von 4 oder 5 % (w/v) und 0,25 M Weinsäure pH 4 für 24 Stunden bei Raumtemperatur gelagert. Zur Isolierung der RNA werden die Komplexe bestehend aus kationischer Verbindung und Nukleinsäure- für 3 min bei 1530 x g abzentrifugiert und in 300 µl eines Puffers enthaltend 6 M Guanidinhydrochlorid, 1 % (v/v) Nonidet-P40 und 50 mM Tris HCL pH 7,0 aufgenommen. Dann werden 400 µg Proteinase K zugegeben und der Ansatz wird für 10 Minuten bei 40°C inkubiert, mit 300 µl einer Lösung enthaltend 80 % (v/v) Ethanol und 10 % (v/v) Nonidet-P40 versetzt und anschließend auf eine Silica-Membran enthaltende spin-Säule aufgetragen. Die Probe wird mittels Zentrifugation durch die Membran geführt. Die spin-Säule wird einmal mit einem Guanidinthiocyanat- und Ethanol-haltigen Waschpuffer gewaschen und zweimal mit einem NaCl- und Ethanol-haltigen Waschpuffer. Die spin-Säule wird für 3 min bei 20000 x g trockenzentrifugiert. Die RNA wird anschließend in 80 µl RNase-freiem Wasser, das ebenfalls mittels Zentrifugation durch die Membran geführt wird, eluiert. Jeweils 25 µl des Eluates werden auf einem 1,2 %igen Agarose/Formaldehyd-Gel aufgetrennt (Abb. 7).

Zur Kontrolle (K) wird die HeLa RNA direkt in das Plasma pipettiert und nach 10 Sekunden werden 500 µl eines Puffers, der neben 2 % (w/v) Ethandiyl-1,2-bis(dimethyldecylammonium-bromid) noch 0,25 M Weinsäure pH 4 enthält, zugegeben und für weitere 10 min inkubiert. Danach wird die RNA isoliert wie oben beschrieben.

Abbildung 7 zeigt 5 Agarosegele der o.g. Reaktionsansätze. Die Buchstaben und Zahlen über den Gelen geben die Konzentration der jeweiligen Kationischen Verbindungen A bis E an, wobei:
A: o-Xylylen-bis-decyldimethylammonium bromid
B: m-Xylylen-bis-decyldimethylammonium bromid
D: [1,8]-dimethylnaphthaleno,alpha,alpha'-bis-dimethyldecylammonium bromid
E: Ethandiyl-1,2-bis(decyldimethylphosphonium bromid) und
K: Kontrolle
ist.

## Patentansprüche

1. Verfahren zur Stabilisierung und Isolierung von Nukleinsäuren aus einer biologischen Probe, umfassend den folgenden Schritt:
in Kontaktbringen der biologischen Probe mit mindestens einer kationischen Verbindung der Formel (I) 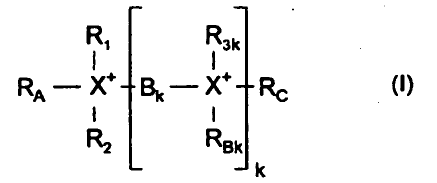 wobei als Anion (A) konjugierte Basen von starken und/oder schwachen anorganischen und/oder organischen Säuren verwendet werden, wobei die Substanz bestehend aus kationischer Verbindung (I) und Anion insgesamt ladungsneutral ist,
und wobei
X Stickstoff (N) oder Phosphor (P) bedeutet,
k die ganzen Zahlen 1, 2, 3, 4, 5, 6, 7, 8, 9, 10, 11, 12, 13, 14, 15, 16, 17, 18, 19, 20, 21, 22, 23 oder 24 bedeutet,
Bₖ bezeichnet aliphatische Alkandiylbrücken, die an keinem, an einem oder an mehreren Kohlenstoff-Atomen substituiert sein können und in denen ein oder mehrere nicht benachbarte Kohlenstoff-Atome durch Sauerstoff ersetzt sein können, der Struktur
-(CH₂)ₙ-(OCH₂)ₘ-
wobei n, m unabhängig voneinander die ganze Zahl 0, 1, 2, 3, 4, 5 oder 6 bedeutet und n + m > 0 ist, alternativ bedeutet
Bₖeine substituierte Phenyl-, Naphthyl- oder Biphenyl-Brücke, die zusätzlich an einem oder an mehreren Kohlenstoff-Atomen substituiert sein kann, der Struktur 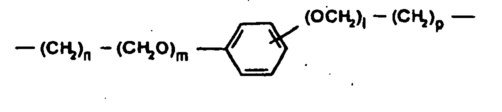 oder 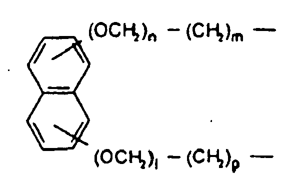 oder 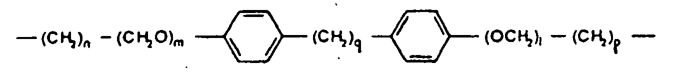 wobei n, m, l, p, q unabhängig voneinander die ganze Zahl 0, 1, 2, 3, 4, 5 oder 6 bedeutet,
R₁, R₂, R₃ₖ, die identisch oder unterschiedlich voneinander sein können und die unsubstituiert oder ar einem oder mehreren Kohlenstoff-Atomen substituiert sein können, bedeuten Wasserstoff, ein lineares oder verzweigtes C₁-C₆-Alkyl, ein lineares oder verzweigtes C₁-C₆-Alkenyl, ein lineares oder verzweigtes C₁-C₆-Alkinyl,
und/oder die Gruppen Phenyl, Benzyl, Phenylethyl. Phenylpropyl, Phenylisopropyl, Phenylisobutyl, Phenoxymethyl, Phenoxyethyl, Phenoxypropyl, Phenoxyisopropyl. Phenoxybutyl, Phenoxyisobutyl und
R_{A}, R_{Bk}, R_{C}, die identisch oder unterschiedlich voneinander sein können und die unsubstituiert oder an einem oder mehreren Kohlenstoff-Atomen substituiert sein können, bedeuten Wasserstoff, ein lineares oder verzweigtes C₁-C₂-Alkyl, ein lineares oder verzweigtes C₁-C₂₁-Alkenyl, ein lineares oder verzweigtes C₁-C₂₁-Alkinyl, oder eine Struktur
CH₃(̵CH₂)ₙ-Z-(CH₂)ₘ-
wobei n die ganze Zahl 0, 1, 2, 3, 4, 5, 6, 7, 8, 9, 10, 11, 12, 13, 14, 15, 16, 17, 18, 19, 20, 21, 22, 23 oder 24 bedeutet und m unabhängig davon die ganze Zahl 1, 2, 3, 4, 5, 6, 7, 8, 9, 10, 11, 12, 13, 14, 15, 16, 17, 18, 19, 20, 21, 22, 23 oder 24 bedeutet
Z eine der Strukturen -O-, -CO-, -CO₂-, -OCO-, -CO-N-, -N-CO-, -O-CO-N-, -N-CO-O-, -S- oder -S-S-bezeichnet,
alternativ bilden R_{A} und R_{C} zusammen einen Rest R_{AC} und somit eine cyclische Struktur 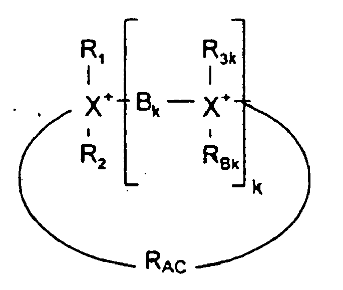 wobei der Rest R_{AC}, der unsubstituiert oder an einem oder mehreren Kohlenstoff-Atomen substituiert sein kann, ein lineares oder verzweigtes C₁-C₈-Alkyl, ein lineares oder verzweigtes C₁-C₈-Alkenyl, ein lineares oder verzweigtes C₁-C₈-Alkinyl bedeutet,
und wobei für k>1 die Brückengruppe Bₖ sowie die Gruppen R_{Bk} und R₃ₖ gleich oder verschieden voneinander sein können.

2. Verfahren nach Anspruch 1, wobei die Verbindungen der allgemeinen Formel (I) verwendet werden mit einem Anion A ausgewählt aus der Gruppe: Fluorid, Chlorid, Bromid, Jodid, Perchlorat, Perbromat, Periodat, Phosphat, Hydrogenphosphat, Dihydrogenphosphat, Sulfat, Thiosulfat, Hydroxid Carbonsäuren, a-Halogen-Carbonsäuren und/oder Hydroxy-Carbonsäuren
und wobei
k bedeutet die ganze Zahl 1, 2, 3, 4, 5 oder 6,
wobei für den Fall, daß Bₖ eine substituierte Phenyl-, Naphthyl- oder Biphenyl-Brücke bezeichnet, n, m, l, p, q unabhängig voneinander die ganze Zahl 0, 1 oder 2 bedeutet,
R₁, R₂, R₃ₖ, die identisch oder unterschiedlich voneinander sein können, bedeuten
die C₁-C₆-Alkyl-Gruppen methyl, ethyl, propyl, isopropyl, butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl und/oder 1-ethyl-2-methyl-propyl,
und/oder die C₃-C₆-Alkenyl-Gruppen 2-propenyl (allyl), 2-butenyl, 3-butenyl, 1-metyl-2-propenyl, 2-methyl-2-propenyl, 2-pentenyl, 3-pentenyl, 4-pentenyl, 1-methyl-2-butenyl, 2-methyl-3-butenyl, 3-methyl-3-butenyl, 1,1-dimethyl-2-propenyl, 1,2-dimethyl-2-propenyl, 1-ethyl-2-propenyl, 2-hexenyl. 3-hexenyl, 4-hexenyl, 5-hexenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-2-butenyl, 1,2-dimethyl-3-butenyl, 1,3-dimethyl-2-butenyl, 1,3-dimethyl-3-butenyl, 2,2-dimethyl-3-butenyl, 2,3-dimethyl-2-ethyl-2-butenyl, 2-ethyl-3-butenyl, 1,1,2-trimethyl-2-propenyl, 1-ethyl-1-methyl-2-propenyl und/oder 1-ethyl-2-methyl-2-propenyl,
und/oder die C₃-C₆-Alkinyl-Gruppen 2-propinyl (propargyl), 2-butinyl, 3-butinyl, 2-pentinyl, 3-pentinyl, 4-pentinyl, 3-methyl-2-butinyl, 2-hexinyl, 3-hexinyl, 4-hexinyl, 5-hexinyl, 3-methyl-2-pentinyl, 4-methyl-2-pentinyl, 2-methyl-3-pentinyl, 4-methyl-3-pentinyl, 1-methyl-4-pentinyl, 1,1-dimethyl-2-butinyl, 1,1-dimethyl-3-butinyl, 1,2-dimethyl-3-butinyl, 1,3-dimethyl-2-butinyl, 2,2-dimethyl-3-butinyl, 1-ethyl-2-butinyl, 1-ethyl-3-butinyl, 2-ethyl-3-butinyl und/oder 1-ethyl-methyl-2-propinyl
und
R_{A}, R_{Bk}, R_{C}, die identisch oder unterschiedlich voneinander sein können, bedeuten
die linearen oder verzweigten C₈-C₂₀-Alkyl-Gruppen octyl, decyl, undecyl, dodecyl, tridecyl, tetradecyl, pentadecyl, hexadecyl, heptadecyl, octadecyl, nonadecyl und/oder eicosyl
und/oder die linearen oder verzweigten C₈-C₂₀-Alkenyl-Gruppen octenyl, decenyl, undecenyl, dodecenyl, tridecenyl, tetradecenyl, pentadecenyl, hexadecenyl, heptadecenyl, octadecenyl, nonadecenyl und/oder eicosenyl
und/oder die linearen oder verzweigten C₈-C₂₀-Alkinyl-Gruppen octinyl, decinyl, undecinyl, dodecinyl, tridecinyl, tetradecinyl, pentadecinyl, hexadecinyl, heptadecinyl, octadecinyl, nonadecinyl und/oder eicosinyl
und/oder eine Struktur
CH₃(̵CH₂)ₙ-Z-(CH₂)ₘ-
wobei n, m unabhängig voneinander sind und n die ganze Zahl 2, 3 oder 4 bedeutet und m die ganze Zahl 8, 9, 10, 11, 12, 13, 14, 15, 16, 17 oder 18 bedeutet und
Z eine der Strukturen -O-, -CO-, -OCO-, -CO-N- oder -N-CO- bezeichnet.

3. Verfahren nach Anspruch 1 oder 2, wobei eine oder mehrere der mit R_{A}, R_{Bk} und R_{C} bezeichneten Gruppen die Struktur
CH₃ - (CH₂)ₙ - O- (CH₂)ₘ - 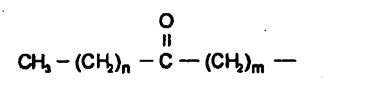 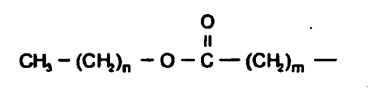 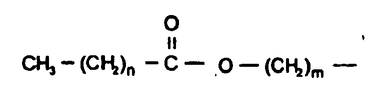 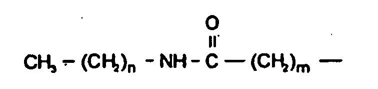
oder 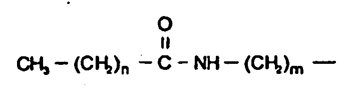 bedeutet.

4. Verfahren nach einem der vorhergehenden Ansprüche, wobei R₁, R₂ und/oder R₃ₖ die Allyl-Gruppe bedeuten.

5. Verfahren nach einem der vorhergehenden Ansprüche, wobei kationische Verbindungen der allgemeinen Formel (I) verwendet werden mit einem Anion A ausgewählt aus der Gruppe: Bromid, Jodid, Perchlorat, Hydrogenphosphat, Sulfat, Acetat, Trifluoroacetat, Trichloroacetat, Benzoat, Oxalat, Succinat, Phthalat, Citrat, Tartrat, Maleat. Malonat, Fumarat
und wobei
k die ganze Zahl 1oder 2 bedeutet,
Bₖ bezeichnet die aliphatischen C₂-C₄-Alkandiylbrücken Ethan-1,1-diyl, Ethan-1,2-diyl. Propan-1,1-diyl. Propan-1,2-diyl, Propan-1,3-diyl, Butan-1,1-diyl, Butan-1,2-diyl, Butan-1,3-diyl und/oder Butan-1,4-diyl, R₁, R₂, R₃ₖ bedeuten methyl, ethyl oder hydroxyethyl,
R_{A}, B_{Bk}, R_{C} bedeuten die linearen C₈-C₂₀-Alkyl-Gruppen octyl, decyl, undecyl, dodecyl, tridecyl, tetradecyl, pentadecyl, hexadecyl, heptadecyl, octadecyl, nonadecyl und/oder eicosyl.

6. Verfahren nach einem der vorhergehenden Ansprüche, wobei R₁, R₂ und R₃ₖ identisch sind und/oder R_{A}, R_{Bk} und R_{C} identisch sind und/oder für k > 1 die Brückengruppen Bₖ identisch sind.

7. Verfahren nach einem der vorhergehenden Ansprüche, wobei die Kohlénstoff-Atome der Gruppen R₁, R₂, R₃ₖ, R_{A}, R_{Bk} und R_{C} mit einem oder mehreren Halogenatomen, insbesondere einem oder mehreren Fluor-Atomen, und/oder einer oder mehreren primären, sekundären und/oder tertiären Hydroxylgruppen und/oder einer oder mehreren -SH, -NH₂, -NH- und/oder =N- Gruppen substituiert sind, wobei die Substituenten untereinander identisch oder nicht identisch sein können.

8. Verfahren nach Anspruch 7, wobei ein oder mehrere der Kohlenstoff-Atome der Gruppen R₁, R₂, R₃ₖ, R_{A}, R_{Bk} und R_{C}, die nicht direkt an eines der Stickstoff-Atome oder Phosphor-Atome der Verbindung 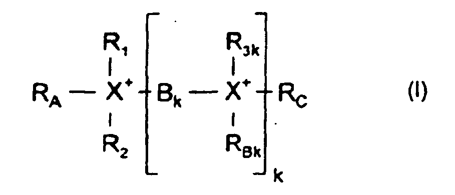 gebunden sind, substituiert sind.

9. Verfahren nach einem der vorhergehenden Ansprüche, wobei die aliphatischen und/oder aromatischen Kohlenstoff-Atome der Brückengruppen Bₖ mit einem oder mehreren Halogenatomen, insbesondere Fluor-Atomen, und/oder einer oder mehreren primären, sekundären und/oder tertiären Hydroxylgruppen und/oder einer oder mehreren -SH, -NH₂, -NH- und/oder =N- Gruppen und/oder einer oder mehreren linearen oder verzweigten C₁-C₄-Alkylgruppen substituiert sind, wobei die Substituenten untereinander identisch oder nicht identisch sein können.

10. Verfahren nach Anspruch 9, wobei ein oder mehrere der aliphatischen und/oder aromatischen Kohlenstoff-Atome der Brückengruppen Bₖ mit einer oder mehreren Methyl-, Ethyl-, Propyl-, i-Propyl-, Butyl-, 2-Methylpropyl- und/oder tert.-Butyl-Gruppe substituiert sind.

11. Verfahren nach einem oder mehreren der vorstehenden Ansprüche, wobei als kationische Verbindung Ethandiyl-1,2-bis(dimethyldecylammoniumbromid) verwendet wird.

12. Verfahren nach einem oder mehreren der Ansprüche 1 bis 10, wobei als kationische Verbindung Propandiyl-1,2-bis(dimethyldecylammoniumbromid) verwendet wird.

13. Verfahren nach einem oder mehreren der Ansprüche 1 bis 10, wobei als kationische Verbindung Ethandiyl-1,2-bis(dimethyltetradecylammoniumbromid) verwendet wird.

14. Verfahren nach einem oder mehreren der vorstehenden Ansprüche 1 bis 10, wobei als kationische Verbindung N,N',N"-Tridecyl-N,N,N',N",N"-pentamethyl-bis-(2-ammonioethyl)ammoniumbromid verwendet wird.

15. Verfahren nach einem der vorstehenden Ansprüche, wobei die mindestens eine kationische Verbindung der Probe in Form eines Feststoffs zugegeben wird.

16. Verfahren nach einem der Ansprüche 1 bis 14, wobei die mindestens eine kationische Verbindung der Probe in Form einer Lösung zugegeben wird, wobei bevorzugt mindestens 0,001 Volumen, weiterhin bevorzugt mindestens 0,01 Volumen, besonders bevorzugt mindestens 0,05 Volumen und insbesondere 1 Volumen der Lösung der Probe zugegeben werden.

17. Verfahren nach Anspruch 16, wobei die Lösung der mindestens einen kationischen Verbindung eine Konzentration von 0,01% bis Sättigung, bevorzugt 0,5 bis 5%, besonders bevorzugt 2 bis 4% aufweist.

18. Verfahren nach einem der vorstehenden Ansprüche, wobei das Verfahren weiterhin den folgenden Schritt aufweist:
- Vermischen der mindestens einen kationischen Verbindung mit der biologischen Probe.

19. Verfahren nach einem der vorstehenden Ansprüche, wobei zur direkten Isolierung von Nukleinsauren nach dem Vermischen inkubiert, bevorzugt 10 Minuten bei Raumtemperatur inkubiert wird.

20. Verfahren nach einem der vorstehenden Ansprüche, wobei der mindestens einen kationischen Verbindung und/oder dem gebildeten Komplex aus Nukleinsäure und kationischer Verbindung(en) weitere Mittel zur Unterstützung der Lyse und/oder Mittel zur Homogenisierung und/oder Mittel zur mechanischen Einwirkung und/oder Mittel zur enzymatischen Einwirkung zugefügt werden.

21. Verfahren nach Anspruch 20, wobei die Mittel zur Unterstützung der Lyse Alkohole, insbesondere verzweigte oder unverzweigte C1- bis C4- Alkanole, wie Isopropanol, Aldehyde, insbesondere niedere C1- bis C4-Aldehyde, verzweigt oder unverzweigt, wie z. B. Glyocal, Phenole, Phenolderivate, ionische, zwitterionische und nichtionische Detergentien, Sulfydryl reduzierende Reagenzien, insbesondere Dithiothreitol, Phosphorsäurederivate, insbesondere Tributylphosphat, chaotrope Reagenzien wie Harnstoff, Carbonsäuren, wie z. B. Zitronensäure oder Malonsäure, oder einfache Salze, wie Ammoniumsalze oder Alkaliphosphate, sind, die einzeln oder in Kombination vorliegen können.

22. Verfahren nach einem der vorstehenden Ansprüche, wobei die gebildeten Komplexe aus Nukleinsäure und kationischer Verbindung(en) durch Zentrifugation sedimentiert werden.

23. Verfahren nach Anspruch 22, wobei die Zentrifugation bei niedrigen g-Zahlen, insbesondere 500 bis 5000 g, für 3 bis 10 Minuten durchgeführt wird.

24. Verfahren nach Anspruch 22 und/oder 23, wobei der Komplex in dem gebildeten Sediment in einem Puffer aufgelöst wird.

25. Verfahren nach einem der Ansprüche 1 bis 24, wobei die Komplexe aus Nukleinsäure und kationischer Verbindung(en) durch Vakuum, Überdruck, Zentrifugation oder Kapillarkräfte auf der Oberfläche einer Membran konzentriert werden.

26. Verfahren nach Anspruch 25, wobei die auf der Oberfläche einer Membran konzentrierten Komplexe zur Freisetzung der Nukleinsäuren aufgelöst und die freigesetzten Nukleinsäuren wiederum auf einer Membran, bevorzugt derselben Membran, gebunden werden.

27. Verfahren nach einem der vorstehenden Ansprüche, wobei die biologische Probe eine Lebensmittelprobe ist, die freie oder gebundene Nukleinsäuren oder nukleinsaurehaltige Zellen enthält, eine Umweltprobe ist, die frei oder gebundene Nukleinsäuren oder nukleinsäurehaltige Zellen enthält, ein zellfreies Probematerial, eine Suspension von Zellen, Viren, Bakterien oder Hefen, ein Gewebe jeder Art oder eine klinische Probe, wie Blut, Plasma, Serum, Sperma, Sputum, Urin, Faeces, Leukozytenfraktionen, Crusta Phlogistica oder Abstriche, sowie eine Pflanze oder ein Pflanzenteil oder freie Nukleinsäuren sein kann.

28. Verfahren nach Anspruch 1, umfassend nach dem Schritt des Inkontaktbringens der biologischen Probe mit der (den) kationischen Verbindung(en) die folgenden weiteren Schritte:
- Gegebenenfalls Zusatz geeigneter Mittel zur Unterstützung der Lyse und/oder zur enzymatischen Einwirkung und/oder mechanischen Einwirkung auf die zusammengeführte Probe/kationische Verbindung(en),
- Mischen der erhaltenen Probe,
- Sammeln der Komplexe aus Nukleinsäure und kationischer Verbindung auf dem Boden eines Gefäßes oder an einer Membran durch Zentrifugation, Vakuum, Uberdruck und/oderKapillarkräfte,
- gegebenenfalls Waschen der Komplexe mit einer geeigneten Waschlösung unter Einsatz von Zentrifugation. Überdruck, Vakuum und/oder Kapillarkräften,
- gegebenenfalls Zugabe einer geeigneten Reagentienlösung,
- Auflösung der Komplexe zur Feisetzung der Nukleinsäuren unter nicht bindenden oder bindenden Bedingungen und
- Isolierung der freigesetzten Nukleinsäure.

29. Verfahren nach einem der Ansprüche 1 bis 28, wobei dieses automatisiert ist.

## Claims

1. A method for stabilization and isolation of nucleic acids from a biological sample, comprising the following step:
bringing the biological sample into contact with at least one cationic compound with the formula (I) 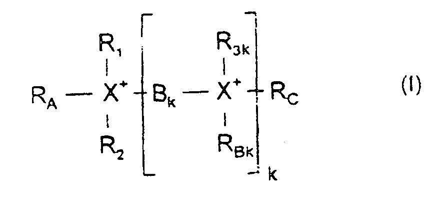
whereas conjugated bases from strong and/or weak inorganic and/or organic acids are used as anion (A), wherein the substance consisting of cationic compound (I) and anion is overall neutral in charge, and wherein
X means nitrogen (N) or phosphor (P),
k means the integers 1, 2, 3, 4, 5, 6, 7, 8, 9, 10, 11, 12, 13, 14, 15, 16, 17, 18, 19, 20, 21, 22, 23 or 24,
Bₖ designates aliphatic alkandiyl bridges, which can be substituted at none, at one or at several carbon atoms and in which one or more carbon atoms, being not adjacent, can be substituted by oxygen, with the structure
- (CH₂)ₙ-(OCH₂)ₘ-
wherein n, m independently from each other mean the integer 0, 1, 2, 3, 4, 5 or 6 and n + m is > 0,
alternatively
Bₖ means a substituted phenyl, naphthyl, or biphenyl bridge, which can additionally be substituted at one or more carbon atoms, with the structure 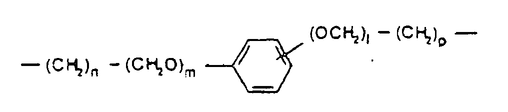 or 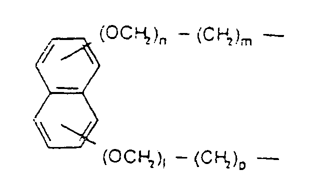 or 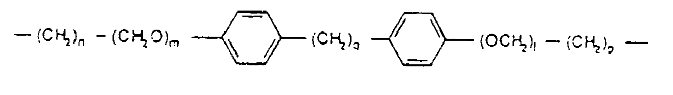 whereas n, m, l, p, q independently from each other mean the integer 0, 1, 2, 3, 4, 5 or 6,
R₁, R₂, R₃ₖ, which can be identical or different from each other or which can be not substituted or substituted at one or more carbon atoms, mean hydrogen, a linear or branched C₁-C₆-alkyl, a linear or branched C₁-C₆-alkenyl, a linear or branched C₁-C₆-alkinyl,
and/or the groups phenyl, benzyl, phenylethyl, phenylpropyl, phenylisopropyl, phenylisobutyl, phenoxymethyl, phenoxyethyl, phenoxypropyl, phenoxyisopropyl, phenoxybutyl, phenoxyisobutyl
and
R_{A}, R_{Bk}, R_{C}, which can be identical or different from each other and which can be not substituted or substituted at one or more carbon atoms, mean hydrogen, a linear or branched C₁-C₂₁-alkyl, a linear or branched C₁-C₂₁-alkenyl, a linear or branched C₁-C₂₁-alkinyl, or a structure
CH₃-(CH₂)ₙ-Z-(CH₂)ₘ-
wherein n means the integer 0, 1, 2, 3, 4, 5, 6, 7, 8, 9, 10, 11, 12, 13, 14, 15, 16, 17, 18, 19, 20, 21, 22, 23 or 24 and irrespective of that m means the integer 1, 2, 3, 4, 5, 6, 7, 8, 9, 10, 11, 12, 13, 14, 15, 16, 17, 18, 19, 20, 21, 22, 23 or 24,
Z designates one of the structures -O-, -CO-, -CO₂-, -OCO-,-CO-N-,-N-CO-,-O-CON-, -N-CO-O-, -S-, or -S-S-,
alternatively R_{A} and R_{C} altogether form a moiety R_{AC} and therefore a cyclic structure 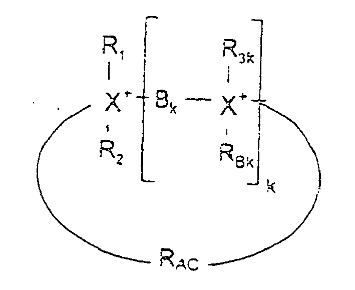
wherein the moiety R_{AC}, which can be not substituted or substituted at one or more carbon atoms, means a linear or branched C₁-C₈-alkyl, a linear or branched C₁-C₈-alkenyl, a linear or branched C₁-C₈-alkinyl,
and wherein for k>1 the bridging group Bₖ as well as the groups R_{Bk} and R₃ₖ can be identical or different from each other.

2. The method according to claim 1, whereby the compounds with the common formula (I) are used with an anion A, selected from the group: fluoride, chloride, bromide, iodide, perchlorate, perbromate, periodate, phosphate, hydrogen phosphate, dihydrogen phosphate, sulfate, thiosulfate, hydroxide, carbonic acids, α-halogen-carbonic acids and/or hydroxy-carbonic acids
and wherein
k means the integer 1, 2, 3, 4, 5 or 6,
wherein in case that Bₖ designates a substituted phenyl, naphthyl or biphenyl bridge, n, m, l, p, q mean the integer 0, 1 or 2 independently from each other,
R₁, R₂, R₃ₖ, which can be identical or different from each other, mean
the C₁-C₆-alkyl groups methyl, ethyl, propyl, isopropyl, butyl, 1-methylpropyl, 2-methylpropyl, 1,1-dimethylethyl, n-pentyl, 1-methylbutyl. 2-methylbutyl, 3-methylbutyl, 1.1-dimethylpropyl, 1,2-dimethylpropyl, 2,2-dimethylpropyl, 1-ethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and/or 1-ethyl-2-methylpropyl,
and/or the C₃-C₆-alkenyl groups 2-propenyl (allyl), 2-butenyl, 3-butenyl, 1-methyl-2-propenyl, 2-methyl-2-propenyl, 2-pentenyl, 3-pentenyl, 4-pentenyl, 1-methyl-2-butenyl, 2-methyl-3-butenyl, 3-methyl-3-butenyl, 1,1-dimethyl-2-propenyl, 1,2-dimethyl-2-propenyl, 1-ethyl-2-propenyl, 2-hexenyl, 3-hexenyl, 4-hexenyl, 5-hexenyl, 1-methyl-2-pentenyl, 2-methyl-2-pentenyl, 3-methyl-2-pentenyl, 4-methyl-2-pentenyl. 1-methyl-3-pentenyl, 2-methyl-3-pentenyl, 3-methyl-3-pentenyl, 4-methyl-3-pentenyl, 1-methyl-4-pentenyl, 3-methyl-4-pentenyl, 4-methyl-4-pentenyl, 1,1-dimethyl-2-butenyl, l.l-dimethyl-3-butenyl, 1.2-dimethyl-2-butenyl, 1,2-dimethyl-3-butenyl, 1,3-dimethy-1-2-butenyl, 1,3-dimethyl-3-butenyl, 2,2-dimethyl-3-butenyl, 2,3-dimethyl-2-ethyl-2-butenyl, 2-ethyl-3-butenyl, 1,1,2-trimethyl-2-propenyl, 1-ethyl-1-methyl-2-propenyl and/or 1-ethyl-2-methyl-2-propenyl,
and/or the C₃-C₆-alkinyl groups 2-propinyl (propargyl), 2-butinyl, 3-butinyl, 2-pentinyl, 3-pentinyl, 4-pentinyl, 3-methyl-2-butinyl, 2-hexinyl, 3-hexinyl, 4-hexinyl, 5-hexinyl, 3-methyl-2-pentinyl, 4-methyl-2-pentinyl, 2-methyl-3-pentinyl, 4-methyl-3-pentinyl, 1-methyl-4-pentinyl, 1,1-dimethyl-2-butinyl, 1,1-dimethyl-3-butinyl, 1,2-dimethyl-3-butinyl, 1,3-dimethyl-2-butinyl, 2,2-dimethyl-3-butinyl, 1-ethyl-2-butinyl, 1-ethyl-3-butinyl, 2-ethyl-3-butinyl and/or 1-ethyl-1-methyl-2-propinyl
and
R_{A}, R_{Bk}. R_{C}, which can be identical or different from each other, mean
the linear or branched C₈-C₂₀-alkyl groups octyl, decyl, undecyl, dodecyl, tridecyl, tetradecyl, pentadecyl, hexadecyl, heptadecyl, octadecyl, nonadecyl and/or eicosyl
and/or the linear or branched C₈-C₂₀-alkenyl groups octenyl, decenyl, undecenyl, dodecenyl, tridecenyl, tetradecenyl, pentadecenyl, hexadecenyl, heptadecenyl, octadecenyl, nonadecenyl and/or eicosenyl
and/or the linear or branched C₈-C₂₀-alkinyl groups octinyl, decinyl, undecinyl, dodecinyl, tridecinyl, tetradecinyl, pentadecinyl, hexadecinyl, heptadecinyl, octadecinyl, nonadecinyl and/or eicosinyl
and/or a structure
CH₃-(CH₂)ₙ-Z-(CH₂)ₘ-
wherein n, m are independent from each other and n means the integer 2, 3 or 4 and m means the integer 8, 9, 10, 11, 12, 13, 14, 15, 16, 17 or 18 and
Z designates one of the structures -O-, -CO-, -OCO-, -CO-N- or -N-CO-.

3. The method according to claim 1 or 2, where in one or more of the groups designated with E_{A}, R_{Bk} and R_{C} mean the structure
CH₃-(CH₂)ₙ-O-(CH₂)ₘ- 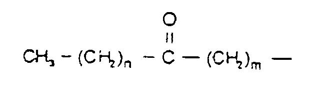 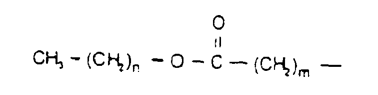 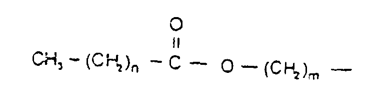 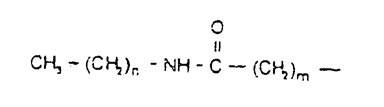
or

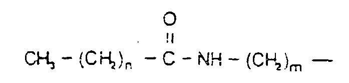

4. The method according to one of the previous claims, whereby R₁, R₂ and/or R₃ₖ mean the allyl group.

5. The method according to one of the previous claims, wherein cationic compounds of the common formula (I) are used with an anion A selected from the group: bromide, iodide, perchlorate, hydrogenphosphate, sulphate, acetate, trifluoroacetate, trichloroacetate, benzoate, oxalate, succinate, phthalate, citrate, tartrate, maleate, malonate, fumarate
and whereas
k means the integer 1 or 2,
Bₖ designates the aliphatic C₂-C₄ alkanidyl bridges ethane-1,1-diyl, ethane-1,2-diyl, propane-1,1-diyl, propane-1,2-diyl, propane-1,3-diyl, butane-1,1-diyl, butane-1,2-diyl, butane-1,3-diyl and /or butane-1,4-diyl,
R₁, R₂, R₃ₖ mean methyl, ethyl or hydroxyethyl,
R_{A}, B_{Bk}, R_{C} mean the linear C₈-C₂₀ alkyl groups octyl, decyl, undecyl, dodecyl, tridecyl, tetradecyl, pentadecyl, hexadecyl, heptadecyl, octadecyl, nonadecyl and/or eicosyl.

6. The method according to one of the previous claims, wherein R₁, R₂ and R₃ₖ are identical and/or R_{A}, R_{Bk} and R_{C} are identical and/or for k>1 the bridging groups Bₖ are identical.

7. The method according to one of the previous claims, wherein the carbon atoms of the groups R₁, R₂, R₃ₖ, R_{A}, R_{Bk} and R_{C} are substituted with one or more halogen atoms, particularly one or more fluorine atoms, and/or one or more primary, secondary and/or tertiary hydroxyl groups and/or one or more -SH, -NH₂, -NH- and/or =N- groups, whereas the substituents can be identical or not identical among each other.

8. The method according to claim 7, wherein one or more of the carbon atoms of the groups R₁, R₂, R₃ₖ, R_{A}, R_{Bk} and R_{C}, which are not directly bound to one of the nitrogen atoms or phosphor atoms of the compound 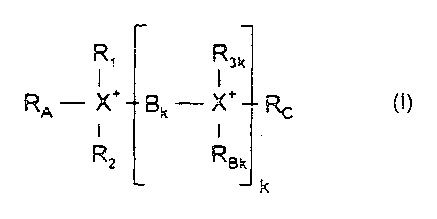 are substituted.

9. The method according to one of the previous claims, wherein the aliphatic and/or aromatic carbon atoms of the bridging groups Bₖ are substituted with one or more halogen atoms, particularly fluorine atoms, and/or one or more primary, secondary and/or tertiary hydroxyl groups and/or one or more -SH, -NH₂, -NH- and/or =N-groups and/or one or more linear or branched C₁-C₄-alkyl groups, whereas the substituents can be identical or not identical among each other.

10. The method according to claim 9, wherein one ore more of the aliphatic and/or aromatic carbon atoms of the bridging group Bₖ are substituted with one or more methyl, ethyl, propyl, i-propyl, butyl, 2-methylpropyl and/or tert, butyl group.

11. The method according to one or more of the previous claims, wherein ethandiyl-1,2-bis(dimethyldecylammoniumbromide) is used as cationic compound.

12. The method according to one or more of the claims 1 to 10, wherein propandiyl-1,2-bis(dimethyldecylammoniumbromide) is used as cationic compound.

13. The method according to one or more of the claims 1 to 10, wherein ethandiyl-1,2-bis(dimethyltetradecylammoniumbromide) is used as cationic compound.

14. The method according to one or more of the previous claims 1 to 10, wherein N,N',N"-tridecyl-N,N,N',N",N"-pentamethyl-bis-(2-ammonioethyl)ammoniumbromide is used as cationic compound.

15. The method according to one of the previous claims, wherein the at least one cationic compound of the sample is added in the form of a solid.

16. The method according to one of the claims 1 to 14, wherein the at least one cationic compound of the sample is added in the form of a solution, whereby preferably at least 0.001 volume, further preferred at least 0.01 volume, particularly preferred at least 0.05 volume and particularly 1 volume of the solution is added to the sample.

17. The method according to claim 16, wherein the solution of the at least one cationic compound has a concentration of 0.01% to saturation, preferably 0.5 to 5%, particularly preferred 2 to 4%.

18. The method according to one of the previous claims, wherein the method further comprises the following step:
- mixing of the at least one cationic compound with the biological sample.

19. The method according to one of the previous claims, wherein incubation is done for direct isolation of nucleic acid after mixing, preferably 10 minutes at room temperature.

20. The method according to one of the previous claims, wherein to the at least one cationic compound and/or the generated complex of nucleic acid and cationic compound(s) further means are added in support of the lysis and/or means for homogenization and/or means for mechanical impact and/or means for enzymatic impact.

21. The method according to claim 20, wherein the means for support of lysis are alcohols, particularly branched or not branched C₁ to C₄ alkanols, such as isopropanole, aldehydes, particularly low C₁ to C₄ aldehydes, branched or not branched, such as glyocal, phenols, phenol derivatives, ionic, zwitterionic and nonionic detergents, sulfydryl reducing reagents, particularly dithiothreitol, phosphoric acid derivatives, particularly tributylphosphate, chaotropic reagents such as urea, carboxylic acids, such as citric acid or malonic acid, or simple salts, such as ammonium salts or alkali phosphates, which can be present separately or in combination.

22. The method according to one of the previous claims, wherein the generated complexes of nucleic acid and cationic compound(s) are sedimented by centrifugation.

23. The method according to claim 22, wherein the centrifugation is performed at low g-values, particularly 500 to 5000 g, for 3 to 10 minutes.

24. The method according to claim 22 and/or 23, wherein the complex in the generated sediment is dissolved in a buffer.

25. The method according to one of the claims 1 to 24, wherein the complexes of nucleic acid and cationic compound(s) are concentrated on the surface of a membrane by vacuum, over pressure, centrifugation or capillary forces.

26. The method according to claim 25, wherein the complexes concentrated on the surface of a membrane are dissolved in order to release the nucleic acids and the released nucleic acids are again fixed on a membrane, preferably on the same membrane.

27. The method according to one of the previous claims, wherein the biological sample is a food sample, which contains free or bound nucleic acids or nucleic acid containing cells, is an environmental probe, containing free or bound nucleic acids or nucleic acid containing cells, can be a cell free sample material, a suspension of cells, viruses, bacteria or yeasts, a tissue of any kind or a clinical sample, such as blood, plasma, serum, sperm, sputum, urine, stool, leucocyte fractions, crusta phlogistica or smears, as well as a plant or part of a plant or free nucleic acids.

28. The method according to claim 1, comprising, after the step of bringing into contact the biological sample with the cationic compound(s), the following further steps:
- optionally addition of appropriate means in support of lysis and/or enzymatic impact and/or mechanical impact on the combined sample/cationic compound(s),
- mixing the obtained sample,
- collecting the complexes generated of nucleic acids and cationic compounds at the bottom of a vessel or at a membrane by centrifugation, vacuum, over pressure and/or capillary forces,
- optionally washing the complexes by an appropriate washing solution using centrifugation, over pressure, vacuum and/or capillary forces,
- optionally addition of an appropriate solution of reagents,
- dissolution of the complexes to release the nucleic acids under non-bonding or bonding conditions and
- isolation of the released nucleic acid.

29. The method according to one of the claims 1 to 28, wherein the method is automated.

## Revendications

1. Procédé de stabilisation et d'isolement d'acides nucléiques à partir d'un échantillon biologique, comprenant l'étape suivante :
mise en contact de l'échantillon biologique avec au moins un composé cationique de formule (I) 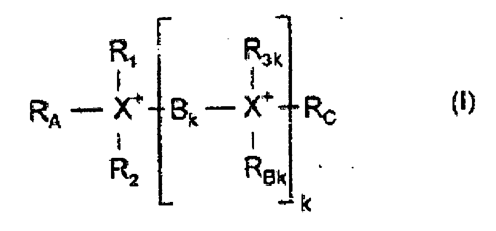
dans laquelle on utilise comme anion (A), des bases conjuguées d'acides minéraux et/ou organiques forts et/ou faibles, la substance constituée du composé cationique (I) et de l'anion étant globalement de charge neutre, et dans lequel X est un atome d'azote (N) ou de phosphore (P),
k désigne les nombres entiers 1, 2, 3, 4, 5, 6, 7, 8, 9, 10, 11, 12, 13, 14, 15, 16, 17, 18, 19, 20, 21, 22, 23 ou 24,
Bₖ désigne des ponts alcane-diyle aliphatiques qui peuvent être substitués au niveau d'aucun, de l'un ou de plusieurs atomes de carbone et dans lesquels un ou plusieurs atomes de carbone non voisins peuvent être remplacés par un atome d'azote, de structure
-(CH₂)ₙ-(OCH₂)ₘ-
dans laquelle, n, m désignent, indépendamment l'un de l'autre, un nombre entier valant 0, 1, 2, 3, 4, 5 ou 6 et n + m > 0,
où
Bₖ désigne un pont phényle, naphtyle ou biphényle qui peut être substitué en outre au niveau d'un ou plusieurs atomes de carbone, de structure 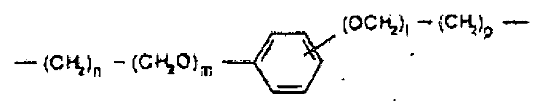 ou 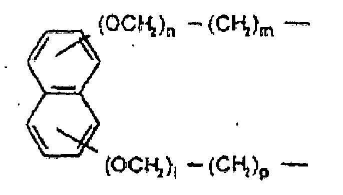 ou 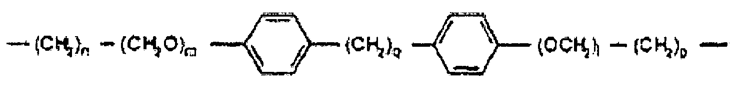
n, m, l, p, q étant indépendamment un nombre entier valant 0, 1, 2, 3, 4, 5 ou 6, R₁, R₂, R₃ₖ, identiques ou différents, non substitués ou substitués sur un ou plusieurs atomes de carbone, désignent un atome d'hydrogène, un groupe alkyle en C₁ à C₆ linéaire ou ramifié, un groupe alcényle en C₁ à C₆ linéaire ou ramifié, un groupe alcinyle en C₁ à C₆ linéaire ou ramifié,
et/ou les groupes phényle, benzyle, phényléthyle, phénylpropyle, phénylisopropyle, phénylisobutyle, phénoxyméthyle, phénoxyéthyle, phénoxypropyle, phénoxyisopropyle, phénoxybutyle, phénoxy-isobutyle et
R_{A}, R_{Bk}, R_{C}, identiques ou différents, non substitués ou substitués sur un ou plusieurs atomes de carbone, désignent un atome d'hydrogène, un groupe alkyle en C₁ à C₂₁ linéaire ou ramifié, un groupe alcényle en C₁ à C₂₁ linéaire ou ramifié, un groupe alcinyle en C₁ à C₂₁ linéaire ou ramifié ou une structure
CH₃(CH₂)ₙ-Z-(CH₂)ₘ-
dans laquelle n désigne un nombre entier valant 0, 1, 2, 3, 4, 5, 6, 7, 8, 9, 10, 11, 12, 13, 14, 15, 16, 17, 18, 19, 20, 21, 22, 23 ou 24, et m indépendamment de celui-ci, désigne un nombre entier valant 1, 2, 3, 4, 5, 6, 7, 8, 9, 10, 11, 12, 13, 14, 15, 16, 17, 18, 19, 20, 21, 22, 23 ou 24,
Z désigne l'une quelconque des structures -O-, -CO-, -CO₂-, -OCO, -CO-N-, -N-CO-, -O-CO-N-, -N-CO-O-, -S- ou -S-S-,
en variante R_{A} et R_{C} forment conjointement un radical R_{AC} et ainsi, une structure cyclique 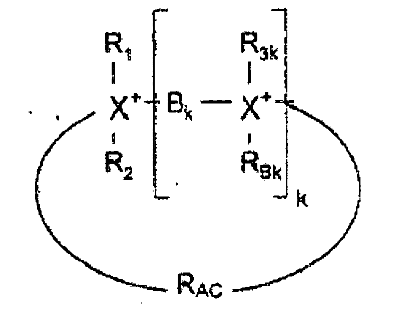
dans laquelle le radical R_{AC} qui peut être non substitué ou substitué au niveau d'un ou plusieurs atomes de carbone, désigne un groupe alkylène en C₁ à C₈ linéaire ou ramifié, un groupe alcénylène en C₁ à C₈ linéaire ou ramifié, un groupe alcinylène en C₁ à C₈ linéaire ou ramifié,
et pour k > 1, les groupes de pontage Bₖ et les groupes R_{Bk} et R₃ₖ pouvant être identiques ou différents les uns des autres.

2. Procédé selon la revendication 1, dans lequel les composés de formule générale (I) sont utilisés avec un anion A choisi dans le groupe formé par le fluorure, le chlorure, le bromure, l'iodure, le perchlorate, le perbromate, le periodate, le phosphate, l'hydrogénophosphate, le dihydrogénophosphate, le sulfate, le thiosulfate, l'hydroxyde, les acides carboxyliques, les α-halogéno-acides carboxyliques et/ou les acides hydroxy-carboxyliques et dans lequel
k désigne un nombre entier valant 1, 2, 3, 4, 5 ou 6,
dans le cas où Bₖ est un pont phényle, naphtyle ou biphényle substitué, n, m, l, p, q indépendamment les uns des autres désignent un nombre entier valant 0, 1 ou 2, R₁, R₂, R₃ₖ, identiques ou différents, désignent, lorsqu'il s'agit de groupes alkyle en C₁ à C₆, des groupes méthyle, éthyle, propyle, isopropyle, butyle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle et/ou 1-éthyl-2-méthylpropyle,
et/ou lorsqu'il s'agit de groupes alcényle en C₃ à C₆, les groupes 2-propényle (allyle), 2-butényle, 3-butényle, 1-méthyl-2-propényle, 2-méthyl-2-propényle, 2-pentényle, 3-pentényle, 4-pentényle, 1-méthyl-2-butényle, 2-méthyl-3-butényle, 3-méthyl-3-butényle, 1,1-diméthyl-2-propnéyle, 1,2-diméthyl-2-propényle, 1-éthyl-2-propényle, 2-hexényle, 3-hexényle, 4-hexényle, 5-hexényle, 1-méthyl-2-pentényle, 2-méthyl-2-pentényle, 3-méthyl-2-pentényle, 4-méthyl-2-pentényle, 1-méthyl-3-pentényle, 2-méthyl-3-pentényle, 3-méthyl-3-pentényle, 4-méthyl-3-pentényle, 1-méthyl-4-pentényle, 3-méthyl-4-pentényle, 4-méthyl-4-pentényle, 1,1-diméthyl-2-butényle, 1,1-diméthyl-3-butényle, 1,2-diméthyl-2-butényle, 1,2-diméthyl-3-butényle, 1,3-diméthyl-2-butényle, 1,3-diméthyl-3-butényle, 2,2-diméthyl-3-butényle, 2,3-diméthyl-2-éthyl-2-butényle, 2-éthyl-3-butényle, 1,1,2-triméthyl-2-propényle, 1-éthyl-1-méthyl-2-propényle et/ou 1-éthyl-2-méthyl-2-propényle,
et/ou lorsqu'il s'agit de groupes alcinyle en C₃ à C₆, les groupes 2-propinyle (propargyle), 2-butinyle, 3-butinyle, 2-pentinyle, 3-pentinyle, 4-pentinyle, 3-méthyl-2-butinyle, 2-hexinyle, 3-hexinyle, 4-hexinyle, 5-hexinyle, 3-méthyl-2-pentinyle, 4-méthyl-2-pentinyle, 2-méthyl-3-pentinyle, 4-méthyl-3-pentinyle, 1-méthyl-4-pentinyle, 1,1-diméthyl-2-butinyle, 1,1-diméthyl-3-butinyle, 1,2-diméthyl-3-butidnyle, 1,3-diméthyl-2-bitunyle, 2,2-diméthyl-3-butinyle, 1-éthyl-2-butinyle, 1-éthyl-3-butinyle, 2-éthyl-3-butinyle et/ou 1-éthyl-1-méthyl-2-propinyle, et
R_{A}, R_{Bk}, R_{C} qui peuvent être identiques ou différents les uns des autres désignent, lorsqu'il s'agit de groupes alkyle en C₈ à C₂₀ linéaires ou ramifiés, les groupes octyle, décyle, undécyle, dodécyle, tridécyle, tétradécyle, pentadécyle, hexadécyle, heptadécyle, octadécyle, nonadécyle et/ou eicosyle,
et/ou, lorsqu'il s'agit de groupes alcényles en C₈ à C₂₀ linéaires ou ramifiés, les groupes octényle, décényle, undécényle, dodécényle, tridécényle, tétradécényle, pentadécényle, hexadécényle, heptadécényle, octadécényle, nonadécényle et/ou eicosényle,
et/ou, lorsqu'il s'agit de groupes alcinyle en C₈ à C₂₀ linéaires ou ramifiés, les groupes octinyle, décinyle, undécinyle, dodécinyle, tridécinyle, tétradécinyle, pentadécinyle, hexandécinyle, heptadécinyle, octadécinyle, nonadécinyle et/ou eicosinyle
et/ou une structure
CH₃(CH₂)ₙ-Z-(CH₂)ₘ-
dans laquelle n, m sont indépendants l'un de l'autre, n désignant le nombre entier 2, 3 ou 4 et m désignant le nombre entier 8, 9, 10, 11, 12, 13, 14, 15, 16, 17 ou 18 et
Z désigne l'une quelconque des structures -O-, -CO-, -OCO-, -CO-N- ou -N-CO-.

3. Procédé selon la revendication 1 ou 2, dans lequel un ou plusieurs des groupes désignés par R_{A}, R_{Bk} et R_{C} ont la structure
CH₃-(CH₂)ₙ-O-(CH₂)ₘ- 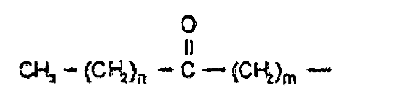 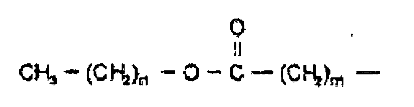 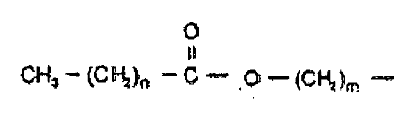 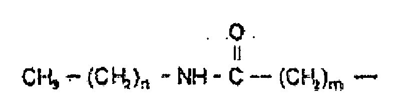
ou

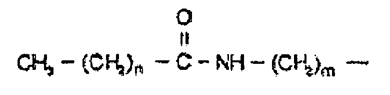

4. Procédé selon l'une quelconque des revendications précédentes, dans lequel R₁, R₂ et/ou R₃ₖ désigne le groupe alkyle.

5. Procédé selon l'une quelconque des revendications précédentes, dans lequel les composés cationiques de formule générale (I) sont utilisés avec un anion A choisi dans le groupe formé par: le bromure, l'iodure, le perchlorate, l'hydrogénophosphate, le sulfate, l'acétate, le trifluoro-acétate, le trichloro-acétate, le benzoate, l'oxalate, le succinate, le phtalate, le citrate, le tartrate, le maléate, le malonate, le fumarate et dans lequel
k désigne le nombre entier 1 ou 2,
Bₖ désigne , en tant que ponts alcane-diyle en C₂ à C₄ aliphatiques, les groupes éthane-1,1-diyle, éthane-1,2-diyle, propane-1,1-diyle, propane-1,2-diyle, propane-1,3-diyle, butane-1,1-diyle, butane-1,2-diyle, butane-1,3-diyle et/ou butane-1,4-diyle, R₁, R₂, R₃ₖ désignent les groupes méthyle, éthyle ou hydroxyéthyle, R_{A}, R_{Bk}, R_{C} désignent, en tant que groupes alkyle en C₈ à C₂₀ linéaires, les groupes octyle, décyle, undécyle, dodécyle, tridécyle, tétradécyle, pentadécyle, hexadécyle, heptadécyle, octadécyle, nonadécyle et/ou eicosyle.

6. Procédé selon l'une quelconque des revendications précédentes dans lequel R₁, R₂ et R₃ₖ sont identiques et/ou R_{A}, R_{Bk} et R_{C} sont identiques et/ou pour k > 1, les groupes de pontage Bₖ sont identiques.

7. Procédé selon l'une quelconque des revendications précédentes, dans lequel les atomes de carbone des groupes R₁, R₂, R₃ₖ, R_{A}, R_{Bk} et R_{C} sont substitués par un ou plusieurs atomes d'halogène, en particulier un ou plusieurs atomes de fluor et/ou un ou plusieurs groupes hydroxyle primaires, secondaires et/ou tertiaires et/ou un ou plusieurs groupes -SH, -NH₂- et/ou =N-, les substituants pouvant être identiques ou différents les uns des autres.

8. Procédé selon la revendication 7, dans lequel un ou plusieurs des atomes de carbone des groupes R₁, R₂, R₃ₖ, R_{A}, R_{Bk} et R_{C} qui ne sont pas directement liés à un atome d'azote ou un atome de phosphore du composé 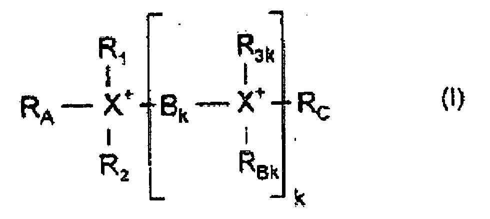 sont substitués.

9. Procédé selon l'une quelconque des revendications précédentes, dans lequel les atomes de carbone aliphatiques et/ou aromatiques des groupes de pontage Bₖ sont substitués par un ou plusieurs atomes d'halogène, en particulier des atomes de fluor et/ou un ou plusieurs groupes hydroxyle primaires, secondaires et/ou tertiaires et/ou ou plusieurs groupes -SH, -NH₂, -NH- et/ou =N- et/ou un ou plusieurs groupes alkyle en C₁ à C₄ linéaires ou ramifiés, les substituants pouvant être identiques ou différents les uns des autres.

10. Procédé selon la revendication 9, dans lequel un ou plusieurs atomes de carbone aliphatiques et/ou aromatiques des groupes de pontage Bₖ sont substitués par un ou plusieurs groupes méthyle, éthyle, propyle, i-propyle, butyle, 2-méthylpropyle et/ou tert-butyle.

11. Procédé selon une ou plusieurs des revendications précédentes, dans lequel on utilise comme composé cationique, l'éthane-diyl-1,2-bis(diméthyldécylammonium-bromure).

12. Procédé selon une ou plusieurs des revendications 1 à 10, dans lequel on utilise comme composé cationique, le propane-diyl-1,2-bis(bromure de diméthyldécylammonium).

13. Procédé selon une ou plusieurs des revendications 1 à 10, dans lequel on utilise comme composé cationique, l'étane-diyl-1,2-bislbromure de diméthyltétradécylammonium).

14. Procédé selon une ou plusieurs des revendications 1 à 10, dans lequel on utilise comme composé cationique, le bromure de N,N',N"-tridécyl-N,N,N',N",N"-pentaméthyl-bis(2-ammonioéthyl)ammonium.

15. Procédé selon une ou plusieurs des revendications précédentes, dans lequel au moins un composé cationique est ajouté à l'échantillon sous la forme d'un solide.

16. Procédé selon une ou plusieurs des revendications 1 à 14, dans lequel au moins un composé cationique est ajouté à l'échantillon sous la forme d'une solution, la solution ajoutée à l'échantillon l'étant de préférence au moins en un volume de 0,001, de manière davantage préférée, un volume d'au moins 0,01, de manière particulièrement préférée, un volume d'au moins 0,05 et en particulier, 1 volume.

17. Procédé selon la revendication 16, dans lequel la solution d'au moins un composé cationique présente une concentration allant de 0,01 % à la saturation, de préférence de 0,5 à 5 %, de manière particulièrement préférée, de 2 à 4 %.

18. Procédé selon l'une quelconque des revendications précédentes, dans lequel le procédé présente en outre l'étape suivante :
- mélange d'au moins un composé cationique avec l'échantillon biologique.

19. Procédé selon l'une quelconque des revendications précédentes, dans lequel pour l'isolement direct d'acides nucléiques, on incube après mélange, de préférence pendant 10 minutes à température ambiante.

20. Procédé selon l'une quelconque des revendications précédentes, dans lequel on ajoute au composé cationique et/ou au complexe formé d'acide nucléique et de composé(s) cationique(s), d'autres agents de stimulation de la lyse et/ou agents d'homogénéisation et/ou agents de traitement mécanique et/ou agent de traitement enzymatique.

21. Procédé selon la revendication 20, dans lequel les agents de stimulation de la lyse sont des alcools, en particulier des alcanols en C₁ à C₄ ramifiés ou non ramifiés tels qu'isopropanol, des aldéhydes, en particulier des aldéhydes en C₁ à C₄ inférieurs, ramifiés ou non ramifiés tels que par exemple le glyocal, des phénols, des dérivés phénoliques, des détergents ioniques, zwitterioniques et non ioniques, des réactifs de réduction des sulfhydryles, en particulier le dithiothréitol, des dérivés d'acide phosphorique, en particulier le phosphate de tributyle, des réactifs chaotropes tels que l'urée, des acides carboxyliques tels que par exemple, l'acide citrique ou l'acide malonique ou des sels simples tels que sel d'ammonium ou alcaliphosphate, qui peuvent se présenter individuellement ou en combinaison.

22. Procédé selon l'une quelconque des revendications précédentes, dans lequel les complexes formés d'acides nucléiques et de composé(s) cationiques sont sédimentés par centrifugation.

23. Procédé selon la revendication 22, dans lequel la centrifugation est réalisée à un nombre de g faible, en particulier à 500 à 5000 g, pendant 3 à 10 minutes.

24. Procédé selon la revendication 22 et/ou 23, dans lequel le complexe dans le sédiment formé est dissous dans un tampon.

25. Procédé selon l'une quelconque des revendications 1 à 24, dans lequel les complexes d'acide nucléique et de composé(s) cationique(s) sont concentrés, à la surface d'une membrane, par application de vide, de surpression, par centrifugation ou forces capillaires.

26. Procédé selon la revendication 25, dans lequel les complexes concentrés sur la surface d'une membrane sont dissous pour libérer les acides nucléiques et les acides nucléiques libérés sont à nouveau liés sur une membrane, de préférence la même membrane.

27. Procédé selon l'une quelconque des revendications précédentes, dans lequel l'échantillon biologique est un échantillon d'aliment qui contient des acides nucléiques libres ou liés ou des cellules contenant des acides nucléiques, un échantillon environnemental qui contient des acides nucléiques libres ou liés ou des cellules contenant des acides nucléiques, un matériau d'échantillon dépourvu de cellules, une suspension de cellules, de virus, de bactéries ou de levures, un tissu de toutes natures ou un échantillon clinique tel que sang, plasma, sérum, sperme, expectoration, urine, fèces, fractions de leucocytes, croûtes phlogistiques ou frottis et une plante ou une partie de plante ou des acides nucléiques libres.

28. Procédé selon la revendication 1, comprenant, après l'étape de mise en contact de l'échantillon biologique avec le/les composé(s) cationique(s), les autres étapes suivantes :
- éventuellement, addition d'agents appropriés pour stimuler la lyse et/ou l'effet enzymatique et/ou l'effet mécanique sur l'échantillon/compose(s) cationique(s) joints,
- mélange de l'échantillon obtenu,
- recueillement du complexe d'acide nucléique et de composé cationique dans le fond d'un récipient ou sur une membrane par centrifugation, application de vide, de surpression et/ou de forces capillaires,
- éventuellement, lavage des complexes avec une solution de lavage appropriée par centrifugation, application de surpression, de vide et/ou de forces capillaires,
- éventuellement, addition d'une solution réactionnelle appropriée,
- dissolution des complexes pour libérer les acides nucléiques dans des conditions non liantes ou liantes et
- isolement des acides nucléiques libérés.

29. Procédé selon l'une quelconque des revendications 1 à 28, dans lequel ces opérations sont automatiques.
